# EUROPEAN PATENT APPLICATION

(11) **EP 3 950 690 A1**
(43) Date of publication of application: **09.02.2022**
(21) Application number: 20783525.7
(22) Date of filing: 27.03.2020
(51) Int. Cl.: C07D 487/04, A61K 31/4188, A61K 31/519, A61P 35/00, A61P 29/00

(54) **PYRROLOHETEROCYCLIC DERIVATIVE, PREPARATION METHOD THEREFOR, AND APPLICATION THEREOF IN MEDICINE**

(30) Priority: 29.03.2019 CN 201910247297; 19.03.2020 CN 202010194720
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); CAI, Guodong, Shanghai 200245 (CN); YANG, Fang, Shanghai 200245 (CN); HE, Feng, Shanghai 200245 (CN); TAO, Weikang, Shanghai 200245 (CN)
(74) Representative: Viganò, Elena
(86) International application number: PCT/CN2020/081591
(87) International publication number: WO 2020/200069

(57) **Abstract**

The present application provides a pyrroloheterocyclic derivative, a preparation method therefor, and an application thereof in medicine. Specifically, the present application provides a novel pyrroloheterocyclic derivative as represented by formula (I), a preparation method therefor, a pharmaceutical composition comprising the derivative, and an application of the derivative as a therapeutic agent, particularly as an ERK inhibitor, wherein substituents in the formula have the same definitions as those in the description.

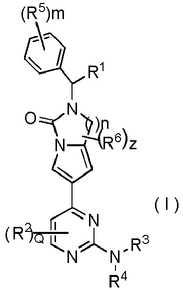

## Description

### TECHNICAL FIELD

The present disclosure belongs to the field of medicine, and relates to a pyrroloheterocyclic derivative, preparation method thereof, and application thereof in medicine. In particular, the present disclosure relates to a pyrroloheterocyclic derivative of formula (I), a method for preparing the same, a pharmaceutical composition comprising the same, a use thereof as an ERK inhibitor for treating ERK-mediated diseases and disorders or for inhibiting the MAPK-ERK signal pathway.

### BACKGROUND

The proliferation, differentiation, metabolism and apoptosis of normal cells are strictly regulated by cell signal transduction pathways in the body. Mitogen-activated protein kinase (MAPK) plays an extremely important role in the signal transduction pathway, and extracellular signal regulated kinase (ERK) is a member of the MAPK family. Through the RAS-RAF-MEK-ERK step, the exogenous stimulating signal is transmitted to the ERK, and the activated ERK is transferred into the cell nucleus to regulate the activity of transcription factors, thereby regulating the biological functions of cells such as cell proliferation, differentiation and apoptosis, or to involve in the regulation of cell morphology and the redistribution of cytoskeleton by phosphorylation of cytoskeletal components in the cytoplasm.

Mutations in RAS and RAF genes cause the continuous activation of the MAPK-ERK signaling pathway, which promotes malignant transformation and abnormal proliferation of cells, and eventually produces tumors (Roberts PJ et al., Oncogene, 2007, 26(22), 3291-3310). The combination of a MEK inhibitor and a B-RAF inhibitor can further enhance the effect of the B-RAF inhibitor on tumor growth, and can significantly improve the disease-free progression and overall survival rate of melanoma patients with BRAFV600E and V600K mutations (Frederick DT et al., Clinical Cancer Research, 2013.19(5), 1225-1231). Although the combination of B-RAF/MEK inhibitors can inhibit tumors, their efficacy is short-lived. Most patients will develop drug resistance within 2-18 months, and tumors will further deteriorate. The mechanism of resistance to B-RAF/MEK inhibitors is very complex, and is mostly directly related to the reactivation of the ERK signaling pathway (Smalley I et al., Cancer Discovery, 2018, 8(2), 140-142). Therefore, the development of new ERK inhibitors is effective not only for patients with mutations in the MAPK signaling pathway, but also for patients with resistance to B-RAF/MEK inhibitors.

B-RAF/MEK inhibitors not only inhibit tumor growth, but also regulate the immune microenvironment of tumors. B-RAF/MEK inhibitors can enhance the expression of tumor-specific antigens, improve the recognition and killing of tumors by antigen-specific T cells, and promote the migration and infiltration of immune cells. In animal models, after treatment with B-RAF/MEK inhibitors, the expression of PD-L1 in tumor tissues is enhanced. When combined with antibodies to checkpoint molecules (such as PD-1 antibody, CTLA4 antibody), it is more effective in inhibiting tumor growth than B-RAF/MEK inhibitors used alone (Boni A et al., Cancer Research, 2010, 70(13), 5213-5219). Studies have shown that ERK inhibitors are similar to B-RAF/MEK inhibitors, and their combination with checkpoint antibodies can regulate the tumor microenvironment, improve the function of cytotoxic T cells, and achieve the effect of inhibiting tumor growth.

At present, many compounds have been developed. Among them, BVD-523 developed by BioMed Valley Discoveries is in clinical phase II, MK-8353 developed by Merck and Astex-029 developed by Astex are in clinical phase I. Relevant patents include WO1999061440A1, WO2001056557A2, WO2001056993A2, WO2001057022A2, WO2002022601A1, WO2012118850A1, WO2013018733A1, WO2014179154A2, WO2015103133A1, WO2016192063A1, WO2017180817A1, and WO2018049127A1.

### SUMMARY OF THE INVENTION

The object of the present disclosure is to provide a compound of formula (I): 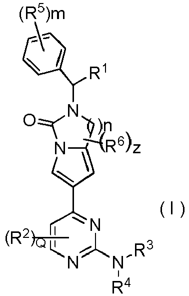
or a stereoisomer, tautomer, mesomer, racemate, enantiomer, diastereomer thereof, or mixture thereof, or a pharmaceutically acceptable salt thereof,
wherein:
   R¹ is selected from the group consisting of hydrogen atom, halogen, alkyl, alkoxy, haloalkyl, haloalkoxy, hydroxy, hydroxyalkyl, cyano, amino, aminoalkyl and nitro, wherein the alkyl is optionally substituted by one or more substituents selected from the group consisting of NR⁷R⁸, alkoxy, halogen, cyano, nitro, hydroxy and hydroxyalkyl;
   each R² is identical or different and each is independently selected from the group consisting of hydrogen atom, halogen, alkyl, alkoxy, haloalkyl, haloalkoxy, hydroxy, hydroxyalkyl, cyano, amino and nitro;
   R³ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
   R⁴ is selected from the group consisting of hydrogen atom, halogen, alkyl, alkoxy, haloalkyl, haloalkoxy, hydroxy, hydroxyalkyl, cyano, amino and nitro;
   each R⁵ is identical or different and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
   R⁶ is selected from the group consisting of hydrogen atom, halogen, alkyl, alkoxy, haloalkyl, haloalkoxy, hydroxy, hydroxyalkyl, cyano, amino and nitro;
   R⁷ and R⁸ are identical or different and are each independently selected from the group consisting of hydrogen atom, alkyl, hydroxyalkyl and haloalkyl;
   m is selected from the group consisting of 0, 1, 2, 3, 4 and 5;
   n is selected from the group consisting of 0, 1, 2 and 3;
   z is selected from the group consisting of 0, 1, 2, 3 and 4; and
   Q is selected from the group consisting of 0, 1 and 2.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R¹ is selected from the group consisting of hydrogen atom, halogen, alkyl, alkoxy, haloalkyl, haloalkoxy, hydroxy, hydroxyalkyl, cyano, amino, aminoalkyl and nitro.

In a preferred embodiment of the present disclosure, the compound of formula (I) is a compound of formula (I-P): 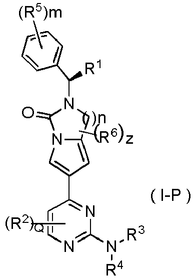
or a stereoisomer, tautomer, mesomer, racemate, enantiomer, diastereomer thereof, or mixture thereof, or a pharmaceutically acceptable salt thereof,
wherein R¹ to R⁶, m, n, z and Q are as defined in formula (I).

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R⁴ is hydrogen atom. In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, n is 1 or 2.

In a preferred embodiment of the present disclosure, the compound of formula (I) is a compound of formula (II): 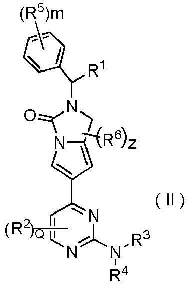
or a stereoisomer, tautomer, mesomer, racemate, enantiomer, diastereomer thereof, or mixture thereof, or a pharmaceutically acceptable salt thereof,
wherein R¹ to R⁶, m, z and Q are as defined in formula (I).

In a preferred embodiment of the present disclosure, the compound of formula (I) is a compound of formula (II-P): 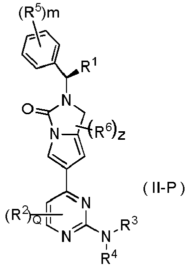
or a stereoisomer, tautomer, mesomer, racemate, enantiomer, diastereomer thereof, or mixture thereof, or a pharmaceutically acceptable salt thereof,
wherein R¹ to R⁶, m, z and Q are as defined in formula (I).

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R¹ is selected from the group consisting of hydrogen atom, alkyl, hydroxy, aminoalkyl, alkylaminoalkyl and hydroxyalkyl; preferably, R¹ is selected from the group consisting of hydrogen atom, C₁₋₆ alkyl, hydroxy, aminoC₁₋₆ alkyl, C₁₋₆ alkylaminoC₁₋₆ alkyl and C₁₋₆ hydroxyalkyl; and more preferably, R¹ is selected from the group consisting of hydrogen atom, methyl, hydroxymethyl, aminomethyl and methylaminomethyl.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R¹ is selected from the group consisting of hydrogen atom, alkyl, hydroxy, aminoalkyl and hydroxyalkyl; preferably, R¹ is selected from the group consisting of hydrogen atom, C₁₋₆ alkyl, hydroxy, aminoC₁₋₆ alkyl and C₁₋₆ hydroxyalkyl; and more preferably, R¹ is selected from the group consisting of hydrogen atom, methyl, hydroxymethyl and aminomethyl.

In a preferred embodiment of the present disclosure, the compound of formula (I) is a compound of formula (III): 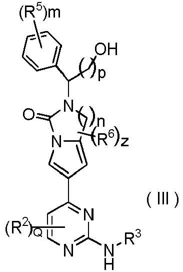
or a stereoisomer, tautomer, mesomer, racemate, enantiomer, diastereomer thereof, or mixture thereof, or a pharmaceutically acceptable salt thereof,
wherein:
   p is selected from the group consisting of 0, 1, 2 and 3, and preferably 1;
   R², R³, R⁵, R⁶, m, n, z and Q are as defined in formula (I).

In a preferred embodiment of the present disclosure, the compound of formula (I) is a compound of formula (III-P): 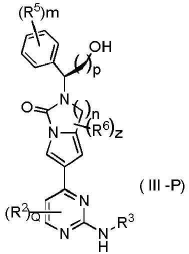
or a stereoisomer, tautomer, mesomer, racemate, enantiomer, diastereomer thereof, or mixture thereof, or a pharmaceutically acceptable salt thereof,
wherein:
   R², R³, R⁵, R⁶, m, n, z, p and Q are as defined in formula (III).

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R² is selected from the group consisting of hydrogen atom, halogen and alkyl; preferably, R² is selected from the group consisting of hydrogen atom, halogen and C₁₋₆ alkyl; and more preferably, R² is C₁₋₆ alkyl.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R³ is selected from the group consisting of alkyl, hydroxyalkyl, cycloalkyl, heterocyclyl and heteroaryl, wherein the alkyl, cycloalkyl, heterocyclyl and heteroaryl are each optionally further substituted by one or more substituents selected from the group consisting of alkyl, alkoxy, oxo, halogen, amino, cyano, nitro, hydroxy and hydroxyalkyl; and preferably, R³ is selected from the group consisting of C₁₋₆ alkyl, C₁₋₆ hydroxyalkyl, C₃₋₆ cycloalkyl, 3 to 8 membered heterocyclyl and 5 to 10 membered heteroaryl, wherein the C₁₋₆ alkyl, C₃₋₆ cycloalkyl, 3 to 8 membered heterocyclyl and 5 to 10 membered heteroaryl are each optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R³ is a heteroaryl, wherein the heteroaryl is optionally further substituted by one or more substituents selected from the group consisting of alkyl, alkoxy, oxo, halogen, amino, cyano, nitro, hydroxy and hydroxyalkyl; preferably, R³ is a 5 to 10 membered heteroaryl, wherein the 5 to 10 membered heteroaryl is optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl; and preferably, R³ is a pyrazolyl, wherein the pyrazolyl is optionally substituted by C₁₋₆ alkyl, and preferably methyl.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R⁵ is selected from the group consisting of hydrogen atom, alkyl, alkoxy and halogen; and preferably, R⁵ is selected from the group consisting of hydrogen atom, C₁₋₆ alkyl, C₁₋₆ alkoxy and halogen.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, R⁶ is a hydrogen atom.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, m is 1 or 2.

In a preferred embodiment of the present disclosure, in the compound of formula (I) or the stereoisomer, tautomer, mesomer, racemate, enantiomer, diastereomer thereof, or mixture thereof, or the pharmaceutically acceptable salt thereof, z is 0 or 1.

Typical compounds of the present disclosure include, but are not limited to:

| Example No. | Structure and name of the compound |
|---|---|
| 1 | 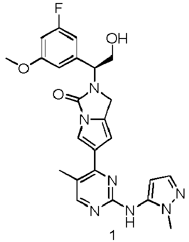 |
| | (*S*)-2-(1-(3-Fluoro-5-methoxyphenyl)-2-hydroxyethyl)-6-(5-methyl-2-((1-methyl-1*H*-pyrazol-5-yl)amino)pyrimidin-4-yl)-1*H*-pyrrolo[1,2-c]imidazol-3(2*H*)-one |
| 2 | 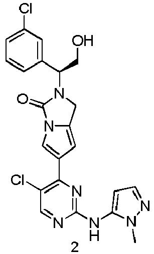 |
| | (*S*)-6-(5-Chloro-2-((1-methyl-1*H*-pyrazol-5-yl)amino)pyrimidin-4-yl)-2-(1 -(3-chlorophenyl)-2-hydroxyethyl)-1*H*-pyrrolo[1,2-*c*]imidazol-3(2*H*)-one |
| 3 | 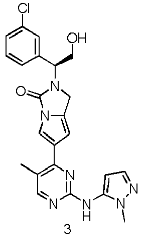 |
| | (*S*)-2-(1-(3-Chlorophenyl)-2-hydroxyethyl)-6-(5-methyl-2-((1-methyl-1*H-*pyrazol-5-yl)amino)pyrimidin-4-yl)-1*H*-pyrrolo[1,2-*c*]imidazol-3(2*H*)-one |
| 4 | 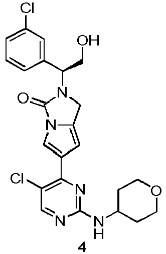 |
| | (*S*)-6-(5-Chloro-2-((tetrahydro-2*H*-pyran-4-yl)amino)pyrimidin-4-yl)-2-(1 -(3-chlorophenyl)-2-hydroxyethyl)-1*H*-pyrrolo[1,2-*c*]imidazol-3(2*H*)-one |
| 5 | 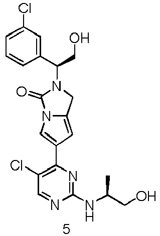 |
| | 6-(5-Chloro-2-(((*S*)-1-hydroxypropan-2-yl)amino)pyrimidin-4-yl)-2-((*S*)-1 -(3-chlorophenyl)-2-hydroxyethyl)-1*H*-pyrrolo[1,2-*c*]imidazol-3(2*H*)-one |
| 6 | 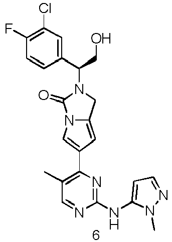 |
| | (*S*)-2-(1-(3-Chloro-4-fluorophenyl)-2-hydroxyethyl)-6-(5-methyl-2-((1-me thyl-1*H*-pyrazol-5-yl)amino)pyrimidin-4-yl)-1*H*-pyrrolo[1,2-*c*]imidazol-3(2 *H*)-one |
| 7 | 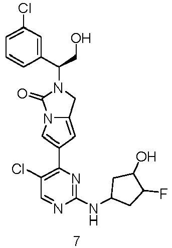 |
| | 6-(5-Chloro-2-((3-fluoro-4-hydroxycyclopentyl)amino)pyrimidin-4-yl)-2-( (*S*)-1-(3-chlorophenyl)-2-hydroxyethyl)-1*H*-pyrrolo[1,2-*c*]imidazol-3(2*H*)-o ne |
| 8 | 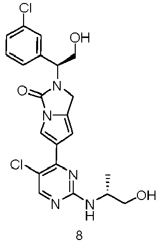 |
| | 6-(5-Chloro-2-(((*R*)-1-hydroxypropan-2-yl)amino)pyrimidin-4-yl)-2-((*S*)-1 -(3-chlorophenyl)-2-hydroxyethyl)-1,2-dihydro-3*H*-pyrrolo[1,2-c]imidazol-3 -one |
| 9 | 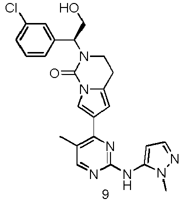 |
| | (*S*)-2-(1-(3-Chlorophenyl)-2-hydroxyethyl)-6-(5-methyl-2-((1-methyl-1*H-*pyrazol-5-yl)amino)pyrimidin-4-yl)-3,4-dihydropyrrolo[1,2-c]pyrimidin-1(2 *H*)-one |
| 10 | 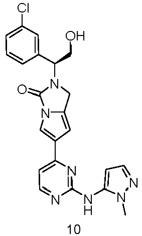 |
| | (*S*)-2-(1-(3-Chlorophenyl)-2-hydroxyethyl)-6-(2-((1-methyl-1*H*-pyrazol-5-yl)amino)pyrimidin-4-yl)-1,2-dihydro-3*H*-pyrrolo[1,2-*c*]imidazol-3-one |
| 11 | 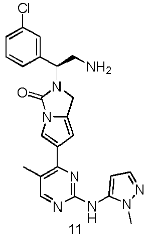 |
| | (*S*)-2-(2-Amino-1-(3-chlorophenyl)ethyl)-6-(5-methyl-2-((1-methyl-1*H*-py razol-5-yl)amino)pyrimidin-4-yl)-1*H*-pyrrolo[1,2-*c*]imidazol-3(2*H*)-one |
| 12 | 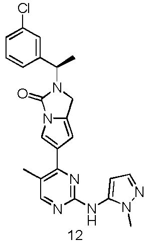 |
| | (*R*)-2-(1-(3-Chlorophenyl)ethyl)-6-(5-methyl-2-((1-methyl-1*H*-pyrazol-5-yl)amino)pyrimidin-4-yl)-1*H*-pyrrolo[1,2-*c*]imidazol-3(2*H*)-one |
| 13 | 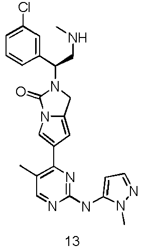 |
| | (*S*)-2-(1-(3-Chlorophenyl)-2-(methylamino)ethyl)-6-(5-methyl-2-((1-meth yl-1*H*-pyrazol-5-yl)amino)pyrimidin-4-yl)-1*H*-pyrrolo[1,2-*c*]imidazol-3(2*H*) -one |
| 14 | 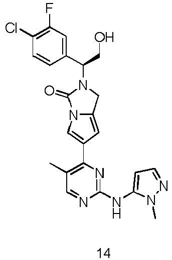 |
| | (*S*)-2-(1-(4-Chloro-3-fluorophenyl)-2-hydroxyethyl)-6-(5-methyl-2-((1-me thyl-1*H*-pyrazol-5-yl)amino)pyrimidin-4-yl)-1*H*-pyrrolo[1,2-*c*]imidazo1-3(2 *H*)-one |
| 15 | 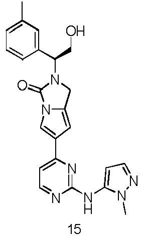 |
| | (*S*)-2-(2-Hydroxy-1-(*m*-tolyl)ethyl)-6-(2-((1-methyl-1*H*-pyrazol-5-yl)amin o)pyrimidin-4-yl)-1*H*-pyrrolo[1,2-*c*]imidazol-3(2*H*)-one |
| 16 | 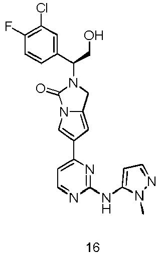 |
| | (*S*)-2-(1-(3-Chloro-4-fluorophenyl)-2-hydroxyethyl)-6-(2-((1-methyl-1*H*-p yrazol-5-yl)amino)pyrimidin-4-yl)-1*H*-pyrrolo[1,2-*c*]imidazol-3(2*H*)-one |
| 17 | 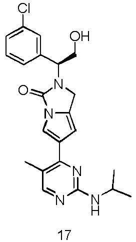 |
| | (*S*)-2-(1-(3-Chlorophenyl)-2-hydroxyethyl)-6-(2-(isopropylamino)-5-meth ylpyrimidin-4-yl)-1*H*-pyrrolo[1,2-*c*]imidazol-3(2*H*)-one |
| 18 | 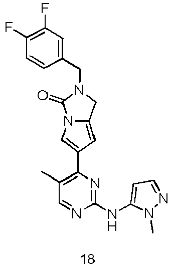 |
| | 2-(3,4-Difluorobenzyl)-6-(5-methyl-2-((1-methyl-1*H*-pyrazol-5-yl)amino) pyrimidin-4-yl)-1*H*-pyrrolo[1,2-c]imidazol-3(2*H*)-one |
| 19 | 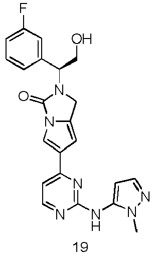 |
| | (*S*)-2-(1-(3-Fluorophenyl)-2-hydroxyethyl)-6-(2-((1-methyl-1*H*-pyrazol-5-yl)amino)pyrimidin-4-yl)-1*H*-pyrrolo[1,2-*c*]imidazol-3(2*H*)-one |
| 20 | 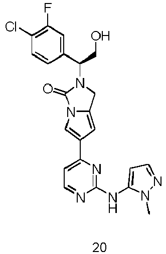 |
| | (*S*)-2-(1-(3-Fluoro-4-chlorophenyl)-2-hydroxyethyl)-6-(2-((1-methyl-1*H*-pyr azol-5-yl)amino)pyrimidin-4-yl)-1*H*-pyrrolo[1,2-*c*]imidazol-3(2*H*)-one |
| 21 | 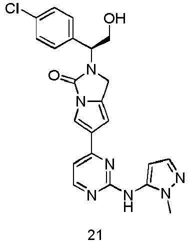 |
| | (*S*)-2-(1-(4-Chlorophenyl)-2-hydroxyethyl)-6-(2-((1-methyl-1*H*-pyrazol-5-yl )amino)pyrimidin-4-yl)-1*H*-pyrrolo[1,2-*c*]imidazol-3(2*H*)-one |

or a stereoisomer, tautomer, mesomer, racemate, enantiomer, diastereomer thereof, or mixture thereof, or a pharmaceutically acceptable salt thereof.

In another aspect, the present disclosure relates to a compound of formula (IIIA): 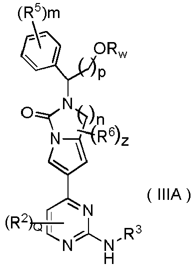
or a stereoisomer, tautomer, mesomer, racemate, enantiomer, diastereomer thereof, or mixture thereof, or a pharmaceutically acceptable salt thereof,
wherein:
   R_{w} is a hydroxy protecting group; and
   R², R³, R⁵, R⁶, m, n, p, Q and z are as defined in formula (III).

In another aspect, the present disclosure relates to a compound of formula (III-PA): 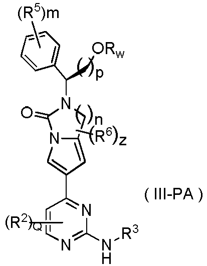
or a stereoisomer, tautomer, mesomer, racemate, enantiomer, diastereomer thereof, or mixture thereof, or a pharmaceutically acceptable salt thereof,
wherein:
   R_{w} is a hydroxy protecting group; and
   R², R³, R⁵, R⁶, m, n, p, Q and z are as defined in formula (III).

Typical compounds of formula (IIIA) of the present disclosure include, but are not limited to:

| Example No. | Structure and name of the compound |
|---|---|
| 1g | 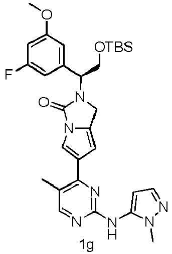 |
| | (*S*)-2-(2-((*tert*-Butyldimethylsilyl)oxy)-1-(3-fluoro-5-methoxyphenyl)ethy l)-6-(5-methyl-2-((1-methyl-1*H*-pyrazol-5-yl)amino)pyrimidin-4-yl)-1*H*-pyr rolo[1,2-*c*]imidazol-3(2*H*)-one |
| 2i | 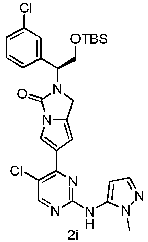 |
| | (*S*)-2-(2-((*tert*-Butyldimethylsilyl)oxy)-1-(3-chlorophenyl)ethyl)-6-(5-chlo ro-2-((1-methyl-1*H*-pyrazol-5-yl)amino)pyrimidin-4-yl)-1*H*-pyrrolo[1,2-*c*]i midazol-3(2*H*)-one |
| 3a | 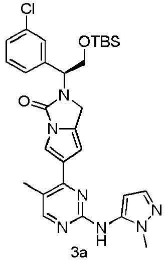 |
| | (*S*)-2-(2-((*tert*-Butyldimethylsilyl)oxy)-1-(3-chlorophenyl)ethyl)-6-(5-met hyl-2-((1-methyl-1*H*-pyrazol-5-yl)amino)pyrimidin-4-yl)-1*H*-pyrrolo[1,2-*c*]i midazol-3(2*H*)-one |
| 5b | 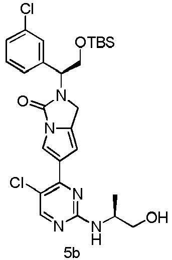 |
| | 2-((*S*)-2-((*tert*-Butyldimethylsilyl)oxy)-1-(3-chlorophenyl)ethyl)-6-(5-chlo ro-2-(((*S*)-1-hydroxypropan-2-yl)amino)pyrimidin-4-yl)-1,2-dihydro-3*H*-pyr rolo[1,2-c]imidazol-3-one |
| 6f | 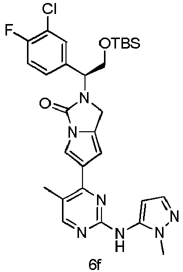 |
| | (*S*)-2-(2-((*tert*-Butyldimethylsilyl)oxy)-1-(3-chloro-4-fluorophenyl)ethyl)-6-(5-methyl-2-((1-methyl-1*H*-pyrazol-5-yl)amino)pyrimidin-4-yl)-1,2-dihyd ro-3*H*-pyrrolo[1,2-*c*]imidazol-3-one |
| 9i | 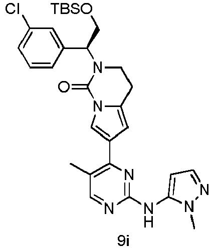 |
| | (*S*)-2-(2-((*tert*-Butyldimethylsilyl)oxy)-1-(3-chlorophenyl)ethyl)-6-(5-met hyl-2-((1-methyl-1*H*-pyrazol-5-yl)amino)pyrimidin-4-yl)-3,4-dihydropyrrol o[1,2-*c*]pyrimidin-1(2*H*)-one |
| 10c | 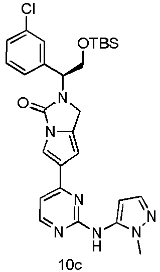 |
| | (*S*)-2-(2-((*tert*-Butyldimethylsilyl)oxy)-1-(3-chlorophenyl)ethyl)-6-(2-((1-methyl-1*H*-pyrazol-5-yl)amino)pyrimidin-4-yl)-1,2-dihydro-3*H*-pyrrolo[1,2-*c*]imidazol-3(2*H*)-one |
| 15f | 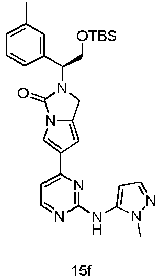 |
| | (*S*)-2-(2-((*tert*-Butyldimethylsilyl)oxy)-1-(*m*-tolyl)ethyl)-6-(2-((1-methyl-1*H*-pyrazol-5-yl)amino)pyrimidin-4-yl)-1*H*-pyrrolo[1,2-*c*]imidazol-3(2*H*)-o ne |
| 17d | 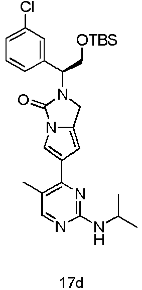 |
| | (*S*)-2-(2-((*tert*-Butyldimethylsilyl)oxy)-1-(3-chlorophenyl)ethyl)-6-(2-(iso propylamino)-5-methylpyrimidin-4-yl)-1*H*-pyrrolo[1,2-*c*]imidazol-3(2*H*)-on e |

or a stereoisomer, tautomer, mesomer, racemate, enantiomer, diastereomer thereof, or mixture thereof, or a pharmaceutically acceptable salt thereof.

In another aspect, the present disclosure relates to a method for preparing the compound of formula (III), comprising a step of: 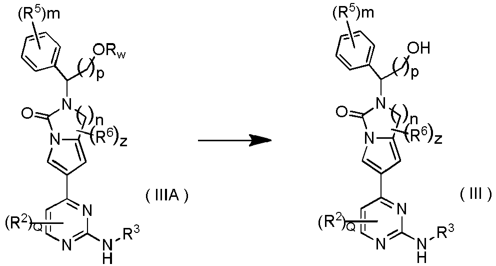
removing the hydroxy protecting group R_{w} from a compound of formula (IIIA) under an acidic condition to obtain the compound of formula (III),
wherein:
   the hydroxy protecting group R_{w} is preferably TBS; and
   R², R³, R⁵, R⁶, m, n, p, Q and z are as defined in formula (III).

In another aspect, the present disclosure relates to a method for preparing the compound of formula (III-P), comprising a step of: 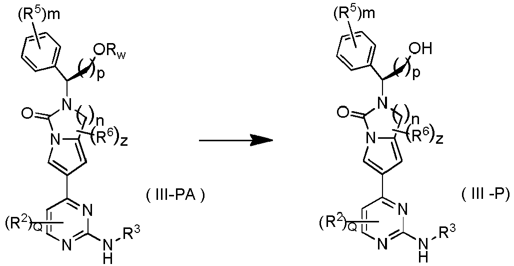
removing the hydroxy protecting group R_{w} from a compound of formula (III-PA) under an acidic condition to obtain the compound of formula (III-P),
wherein:
   the hydroxy protecting group R_{w} is preferably TBS; and
   R², R³, R⁵, R⁶, m, n, p, Q and z are as defined in formula (III).

In another aspect, the present disclosure relates to a pharmaceutical composition, comprising a therapeutically effective amount of the compound of formula (I) or formula (II) or formula (III) or the stereoisomer, tautomer, mesomer, racemate, enantiomer, diastereomer thereof, or mixture thereof, or the pharmaceutically acceptable salt thereof, and one or more pharmaceutically acceptable carriers, diluents or excipients.

The present disclosure further relates to a use of the compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same in the preparation of a medicament for inhibiting ERK.

The present disclosure further relates to a use of the compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same in the preparation of a medicament for the treatment or prevention of cancer, inflammation, or other proliferative diseases, and preferably cancer; wherein the cancer is selected from the group consisting of melanoma, liver cancer, kidney cancer, lung cancer (such as non-small cell lung cancer or small cell lung cancer), nasopharyngeal cancer, colorectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma and glioma.

The present disclosure also relates to a method for inhibiting ERK, comprising a step of administering to a patient in need thereof a therapeutically effective amount of the compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same.

The present disclosure also relates to a method for treating or preventing ERK-mediated diseases, comprising a step of administering to a patient in need thereof a therapeutically effective amount of the compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same.

The present disclosure also relates to a method for treating or preventing cancer, inflammation, or other proliferative diseases, and preferably cancer, comprising a step of administering to a patient in need thereof a therapeutically effective amount of the compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same, wherein the cancer is selected from the group consisting of melanoma, liver cancer, kidney cancer, lung cancer (such as non-small cell lung cancer or small cell lung cancer), nasopharyngeal cancer, colorectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma and glioma.

The present disclosure also relates to a compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same, for use as a medicament.

The present disclosure also relates to a compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same, for use as an ERK inhibitor.

The present disclosure also relates to a compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same, for use in treating or preventing ERK-mediated diseases.

The present disclosure also relates to a compound of formula (I) or formula (II) or formula (III) or the stereoisomer, tautomer, mesomer, racemate, enantiomer, diastereomer thereof, or mixture thereof, or the pharmaceutically acceptable salt thereof or the pharmaceutical composition comprising the same, for use in treating or preventing cancer, inflammation, or other proliferative diseases, and preferably cancer, wherein the cancer is selected from the group consisting of melanoma, liver cancer, kidney cancer, lung cancer (such as non-small cell lung cancer or small cell lung cancer), nasopharyngeal cancer, colorectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma and glioma.

The active compound can be prepared in a form suitable for administration by any appropriate route, and the active compound is preferably in a unit dose form, or in a form in which the patient can self-administer in a single dose. The unit dose of the compound or composition of the present disclosure can be expressed in the form of tablets, capsules, cachets, bottled syrups, powders, granules, lozenges, suppositories, regenerated powders or liquid formulations.

The dosage of the compound or composition used in the treatment method of the present disclosure will generally vary according to the severity of the disease, the weight of the patient, and the relative efficacy of the compound. However, as a general guide, a suitable unit dose can be 0.1 to 1000 mg.

In addition to the active compound, the pharmaceutical composition of the present disclosure can also comprise one or more auxiliaries including a filler (diluent), binder, wetting agent, disintegrant, excipient and the like. Depending on the administration mode, the composition can comprise 0.1 to 99% by weight of the active compound.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can contain one or more ingredient(s) selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets.

An aqueous suspension comprises an active ingredient in admixture with excipients suitable for the manufacture of an aqueous suspension. The aqueous suspension can also comprise one or more preservatives such as ethyl paraben or n-propyl paraben, one or more colorants, one or more flavoring agents, and one or more sweeteners.

An oil suspension can be formulated by suspending the active ingredient in a vegetable oil. The oil suspension can contain a thickener. The aforementioned sweeteners and flavoring agents can be added to provide a palatable formulation.

The active ingredient in admixture with the dispersants or wetting agents, suspending agents or one or more preservatives can be prepared as dispersible powders or granules suitable for the preparation of an aqueous suspension by adding water. Suitable dispersants or wetting agents and suspending agents are exemplified by those already mentioned above. Additional excipients, such as sweeteners, flavoring agents and colorants, can also be added. These compositions can be preserved by adding an antioxidant such as ascorbic acid.

The pharmaceutical composition of the present disclosure can also be in the form of an oil-in-water emulsion.

The pharmaceutical composition can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. For example, the active ingredient is dissolved in a mixture of soybean oil and lecithin. The oil solution is then added into a mixture of water and glycerol, and processed to form a micro-emulsion. The injectable solution or micro-emulsion can be introduced into a patient's bloodstream by local bolus injection. Alternatively, the solution and micro-emulsion are preferably administrated in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium.

The compound of the present disclosure can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug. Such materials include cocoa butter, glycerin gelatin, hydrogenated vegetable oils, mixtures of polyethylene glycols with various molecular weights and fatty acid esters of polyethylene glycols.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### TERM DEDINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, n-propyl, isopropyl, n-butyl, isobutyl, *tert*-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, n-propyl, isopropyl, n-butyl, isobutyl, *tert*-butyl, *sec*-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "cycloalkyl" refers to a saturated or partially unsaturated monocyclic or polycyclic hydrocarbon substituent group having 3 to 20 carbon atoms, preferably 3 to 12 carbon atoms, more preferably 3 to 8 carbon atoms, and most preferably 3 to 6 carbon atoms (for example 3, 4, 5 or 6 carbon atoms). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, preferably cycloalkyl. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

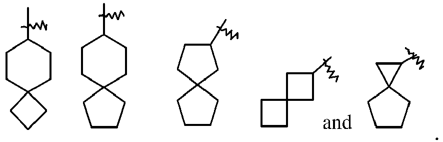

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

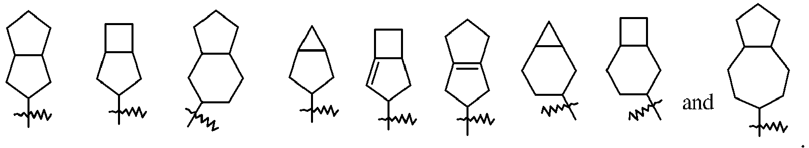

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

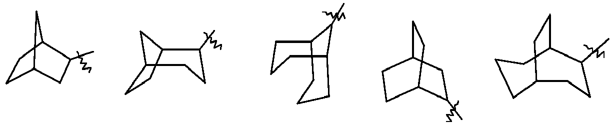

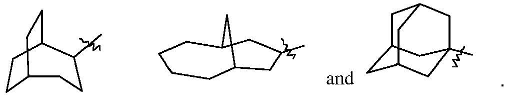

The cycloalkyl (including monocyclic cycloalkyl, spiro cycloalkyl, fused cycloalkyl and bridged cycloalkyl) ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like, and preferably benzocyclopentyl, tetrahydronaphthyl.

The cycloalkyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "alkoxy" refers to an -O-(alkyl) or an -O-(unsubstituted cycloalkyl) group, wherein the alkyl or cycloalkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more groups independently selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "heterocyclyl" refers to a 3 to 20 membered saturated or partially unsaturated monocyclic or polycyclic hydrocarbon substituent group, wherein one or more ring atoms are heteroatoms selected from the group consisting of N, O, S, S(O) and S(O)₂, but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; preferably, 3 to 8 ring atoms wherein 1 to 3 atoms are heteroatoms; and preferably 3 to 6 ring atoms wherein 1 to 3 atoms are heteroatoms. Non-limiting examples of monocyclic heterocyclyl include azetidinyl, pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and the like, and preferably tetrahydropyranyl, piperidinyl, pyrrolidinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O, S, S(O) and S(O)₂, with the remaining ring atoms being carbon atoms, where the rings can contain one or more double bonds, but none of the rings has a completely conjugated π-electron system. The spiro heterocyclyl is preferably a 6 to 14 membered spiro heterocyclyl, and more preferably a 7 to 11 membered spiro heterocyclyl. According to the number of the spiro atoms shared between the rings, the spiro heterocyclyl is divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

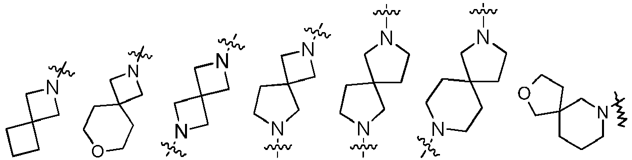

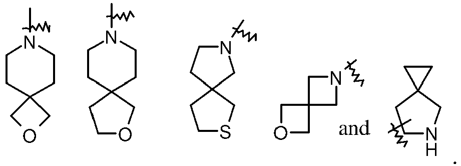

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably a 6 to 14 membered fused heterocyclyl, and more preferably a 7 to 11 membered fused heterocyclyl. According to the number of membered rings, the fused heterocyclyl can be divided into a bicyclic, tricyclic, tetracyclic or polycyclic fused heterocyclyl, and the fused heterocyclyl is preferably a bicyclic or tricyclic fused heterocyclyl, and more preferably a 5-membered/5-membered or 5-membered/6-membered bicyclic fused heterocyclyl. Non-limiting examples of fused heterocyclyl include:

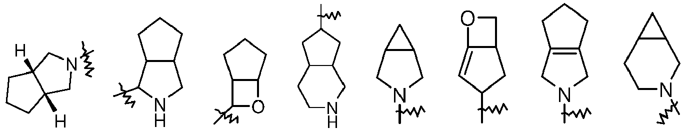

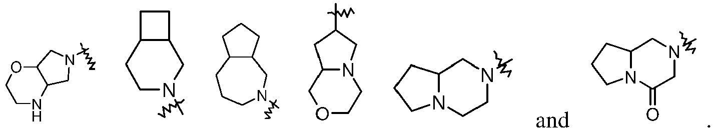

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 11 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

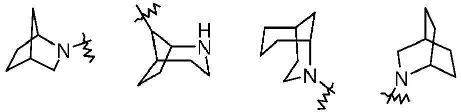

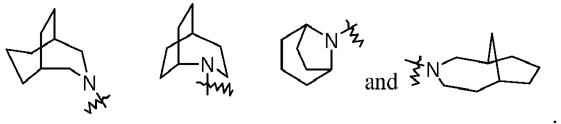

The heterocyclyl (including monocyclic heterocyclyl, spiro heterocyclyl, fused heterocyclyl and bridged heterocyclyl) ring can be fused to the ring of aryl, heteroaryl or cycloalkyl, wherein the ring bound to the parent structure is heterocyclyl. Non-limiting examples thereof include: 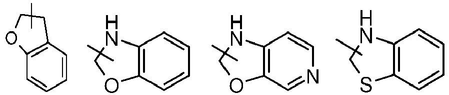 and the like.

The heterocyclyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "aryl" refers to a 6 to 20 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, and more preferably a 6 membered aryl, for example, phenyl and naphthyl. The aryl ring can be fused to the ring of heteroaryl, heterocyclyl or cycloalkyl, wherein the ring bound to the parent structure is aryl ring. Non-limiting examples thereof include:

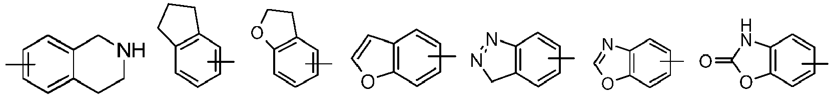

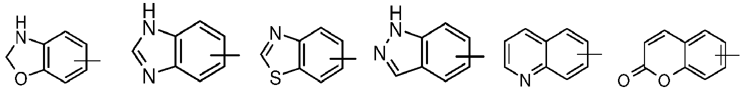

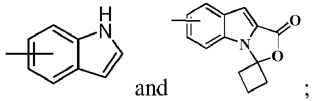

The aryl can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "heteroaryl" refers to a 5 to 20 membered heteroaromatic system having 1 to 4 heteroatoms selected from the group consisting of O, S and N. The heteroaryl is preferably a 5 to 10 membered heteroaryl having 1 to 3 heteroatoms, more preferably a 5 or 6 membered heteroaryl having 1 to 3 heteroatoms. Non-limiting examples include: for example, pyrazolyl, imidazolyl, furyl, thienyl, thiazolyl, oxazolyl, pyrrolyl, triazolyl, tetrazolyl, pyridyl, pyrimidinyl, thiadiazolyl, pyrazinyl and the like. The heteroaryl ring can be fused to the ring of aryl, heterocyclyl or cycloalkyl, wherein the ring bound to the parent structure is heteroaryl ring. Non-limiting examples thereof include:

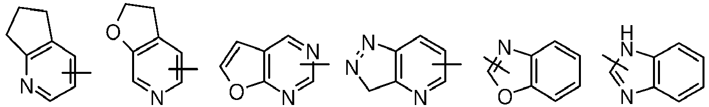

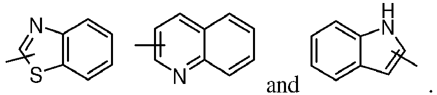

The heteroaryl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyloxy" refers to a -O-cycloalkyl group, wherein the cycloalkyl is as defined above.

The term "haloalkyl" refers to an alkyl group substituted by halogen(s), wherein the alkyl is as defined above.

The term "haloalkoxy" refers to an alkoxy group substituted by halogen(s), wherein the alkoxy is as defined above.

The term "hydroxyalkyl" refers to an alkyl group substituted by hydroxy(s), wherein the alkyl is as defined above.

The term "aminoalkyl" refers to an alkyl group substituted by amino(s), where the alkyl is as defined above.

The term "alkylaminoalkyl" refers to an alkyl group substituted by alkylamino(s), where the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "formyl" refers to a -C(O)H group.

The term "carboxy" refers to a -C(O)OH group.

The term "alkoxycarbonyl" refers to a -C(O)O(alkyl) or -C(O)O(cycloalkyl) group, wherein the alkyl and cycloalkyl are as defined above.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents, wherein each substituent has independent options (that is, the substituents can be identical or different). It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present disclosure, which is safe and effective in mammals and has the desired biological activity.

The compound of the present disclosure can also comprise isotopic derivatives thereof. The term "isotopic derivatives" refers to compounds that differ in structure only in the presence of one or more isotopically enriched atoms. For example, a compound having the structure of the present disclosure except replacing hydrogen with "deuterium" or "tritium", or replacing fluorine with an ¹⁸F-fluorine labeling (¹⁸F isotope), or replacing carbon with ¹¹C-, ¹³C-, or ¹⁴C-enriched carbon (¹¹C-, ¹³C-, or ¹⁴C-carbon labeling; ¹¹C-, ¹³C-, or ¹⁴C-isotope) is within the scope of the present disclosure. Such compounds can be used, for example, as analytical tools or probes in biological assays, or as tracers for *in vivo* diagnostic imaging of disease, or as tracers for pharmacodynamics, pharmacokinetics or receptor studies. Deuterated compounds can generally retain activity comparable to non-deuterated compounds, and when deuterated at certain specific sites, the resulting compounds can achieve better metabolic stability, thereby obtaining certain therapeutic advantages (such as increased *in vivo* half-life or reduced dosage requirements).

For drugs or pharmacologically active agents, the term "therapeutically effective amount" refers to a sufficient amount of a drug or agent that is non-toxic but can achieve the desired effect. The determination of the effective amount varies from person to person, depending on the age and general condition of the recipient, and also on the specific active substance. The appropriate effective amount in a case can be determined by the person skilled in the art according to routine experiments.

### Synthesis Method of the Compound of the Present Disclosure

In order to achieve the object of the present disclosure, the present disclosure applies the following technical solutions:
Scheme I

A method for preparing the compound of formula (III) or the stereoisomer, tautomer, mesomer, racemate, enantiomer, diastereomer thereof, or mixture thereof, or the pharmaceutically acceptable salt thereof according to the present disclosure, comprising the following steps of: 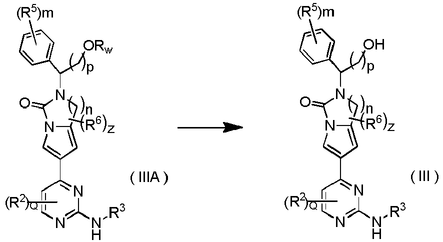
removing the hydroxy protecting group R_{w} from a compound of formula (IIIA) under an acidic condition in a solvent to obtain the compound of formula (III),
wherein:
   the hydroxy protecting group R_{w} is preferably TBS; and
   R², R³, R⁵, R⁶, m, n, p, Q and z are as defined in formula (III).

### Scheme II

In another aspect, the present disclosure relates to a method for preparing the compound of formula (III-P), comprising the following steps of: 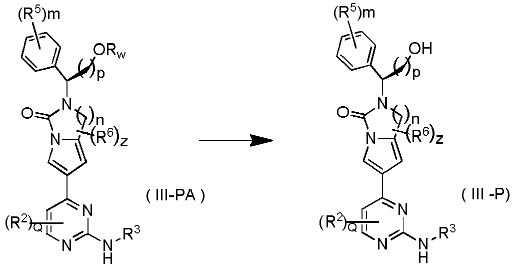
removing the hydroxy protecting group R_{w} from a compound of formula (III-PA) under an acidic condition in a solvent to obtain the compound of formula (III-P),
wherein:
   the hydroxy protecting group R_{w} is preferably TBS; and
   R², R³, R⁵, R⁶, m, n, p, Q and z are as defined in formula (III).

The reagent that provides an acidic condition includes, but is not limited to, hydrogen chloride, trifluoroacetic acid, formic acid, acetic acid, hydrochloric acid, sulfuric acid, methanesulfonic acid, nitric acid, phosphoric acid, *p*-toluenesulfonic acid, Me₃SiCl and TMSOT_{f}; and preferably trifluoroacetic acid.

The reagent that provides an alkaline condition includes organic bases and inorganic bases. The organic bases include, but are not limited to, triethylamine, N,N-diisopropylethylamine, *n*-butyllithium, lithium diisopropylamide, potassium acetate, sodium *tert*-butoxide and potassium *tert*-butoxide. The inorganic bases include, but are not limited to, sodium hydride, potassium phosphate, sodium carbonate, sodium acetate, potassium acetate, potassium carbonate, cesium carbonate, sodium hydroxide, lithium hydroxide and potassium hydroxide.

The deprotection reaction to remove the hydroxy protecting group is well known in the art, and the hydroxy protecting group is, for example, the protecting group described in Protecting Group in Organic Synthesis by T. Greene *et al.* It is generally preferred to use tetrahydropyran-2-yl and *tert*-butyldimethylsilyl as hydroxy protecting groups; and preferably, *tert-* butyldimethylsilyl (TBS).

The hydroxy protecting reagent includes, but is not limited to: methoxymethyl ether, 2-methoxyethoxymethyl ether, tetrahydropyran ether, benzyl ether, p-methoxybenzyl ether, trimethylsilyl ether, triethylsilyl ether, triisopropylsilyl ether, *tert*-butyldimethylsilyl ether, *tert*-butyldimethylchlorosilane, triphenylmethylsilyl ether, acetate, substituted acetate, pivaloate, benzoate, methanesulfonate and p-toluenesulfonate; preferably *tert*-butyldimethylchlorosilane (TBSCl).

The above reactions are preferably carried out in a solvent. The solvent used includes, but is not limited to: acetic acid, methanol, ethanol, n-butanol, toluene, tetrahydrofuran, dichloromethane, petroleum ether, ethyl acetate, n-hexane, dimethyl sulfoxide, 1,4-dioxane, 1,2-dimethoxyethane, water and N,N-dimethylformamide and mixtures thereof; and preferably, dichloromethane.

The details of one or more embodiments of the present disclosure are set forth in the above specification. Although any methods and materials similar or identical to those described herein can be used to perform or test the present disclosure, the preferred methods and materials are described below. Through the specification and claims, other features, purposes and advantages of the present disclosure will be apparent. In the specification and claims, unless the context clearly indicates otherwise, the singular form includes the plural referent. Unless otherwise defined, all technical and scientific terms used herein have the general meanings understood by the person of ordinary skill in the art to which the present disclosure belongs. All patents and publications cited in the specification are incorporated by reference. The following examples are provided to more fully illustrate the preferred embodiments of the present disclosure. These examples should not be construed as limiting the scope of the present disclosure in any way, and the scope of the present disclosure is defined by the claims.

### DETAILED DESCRIPTION

### EXAMPLES

The structures of the compounds are identified by nuclear magnetic resonance (NMR) and/or mass spectrometry (MS). NMR shifts (δ) are given in 10⁻⁶ (ppm). NMR is determined by a Bruker AVANCE-400 machine. The solvents for determination are deuterated-dimethyl sulfoxide (DMSO-*d₆*), deuterated-chloroform (CDCl₃) and deuterated-methanol (CD₃OD), and the internal standard is tetramethylsilane (TMS).

MS is determined by an Agilent 1200 /1290 DAD- 6110/6120 Quadrupole MS liquid chromatograph/mass spectrometer (manufacturer: Agilent, MS model: 6110/6120 Quadrupole MS), waters ACQuity UPLC-QD/SQD (manufacturer: waters, MS model: waters ACQuity Qda Detector/waters SQ Detector), THERMO Ultimate 3000-Q Exactive (manufacturer: THERMO, MS model: THERMO Q Exactive).

High performance liquid chromatography (HPLC) is determined on an Agilent HPLC 1200DAD, Agilent HPLC 1200VWD and Waters HPLC e2695-2489 high pressure liquid chromatograph.

Chiral HPLC is determined on an Agilent 1260 DAD high performance liquid chromatograph.

Preparative chromatography is carried out on Waters 2545-2767, Waters 2767-SQ Detecor2, Shimadzu LC-20AP and Gilson GX-281 preparative chromatographs.

Chiral preparation is carried out on a Shimadzu LC-20AP preparative chromatograph.

CombiFlash rapid preparation instrument used is Combiflash Rf200 (TELEDYNE ISCO).

Yantai Huanghai HSGF254 or Qingdao GF254 silica gel plate is used as the thin layer silica gel chromatography (TLC) plate. The dimension of the silica gel plate used in TLC is 0.15 mm to 0.2 mm, and the dimension of the silica gel plate used in product purification is 0.4 mm to 0.5 mm.

Yantai Huanghai 200 to 300 mesh silica gel is generally used as a carrier for silica gel column chromatography.

The average kinase inhibition rates and IC₅₀ values are determined by a NovoStar microplate reader (BMG Co., Germany).

The known starting materials of the present disclosure can be prepared by the known methods in the art, or can be purchased from ABCR GmbH & Co. KG, Acros Organics, Aldrich Chemical Company, Accela ChemBio Inc., Dari Chemical Company etc.

Unless otherwise stated, the reactions can be carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reaction is performed on a Parr 3916EKX hydrogenation instrument and a Qinglan QL-500 hydrogen generator or HC2-SS hydrogenation instrument.

In hydrogenation reactions, the reaction system is generally vacuumed and filled with hydrogen, and the above operation is repeated three times.

CEM Discover-S 908860 type microwave reactor is used in microwave reactions. Unless otherwise stated, the solution refers to an aqueous solution.

Unless otherwise stated, the reaction temperature is room temperature from 20°C to 30°C.

The reaction process in the examples is monitored by thin layer chromatography (TLC). The developing solvent used in the reactions, the eluent system in column chromatography and the developing solvent system in thin layer chromatography for purification of the compounds include: A: dichloromethane/methanol system, B: n-hexane/ethyl acetate system, and C: petroleum ether/ethyl acetate system. The ratio of the volume of the solvent is adjusted according to the polarity of the compounds, and a small quantity of alkaline reagent such as triethylamine or acidic reagent such as acetic acid can also be added for adjustment.

### Example 1

### (S)-2-(1-(3-Fluoro-5-methoxyphenyl)-2-hydroxyethyl)-6-(5-methyl-2-((1-methyl-1H-py razol-5-yl)amino)pyrimidin-4-yl)-1H-pyrrolo[1,2-c]imidazol-3(2H)-one 1

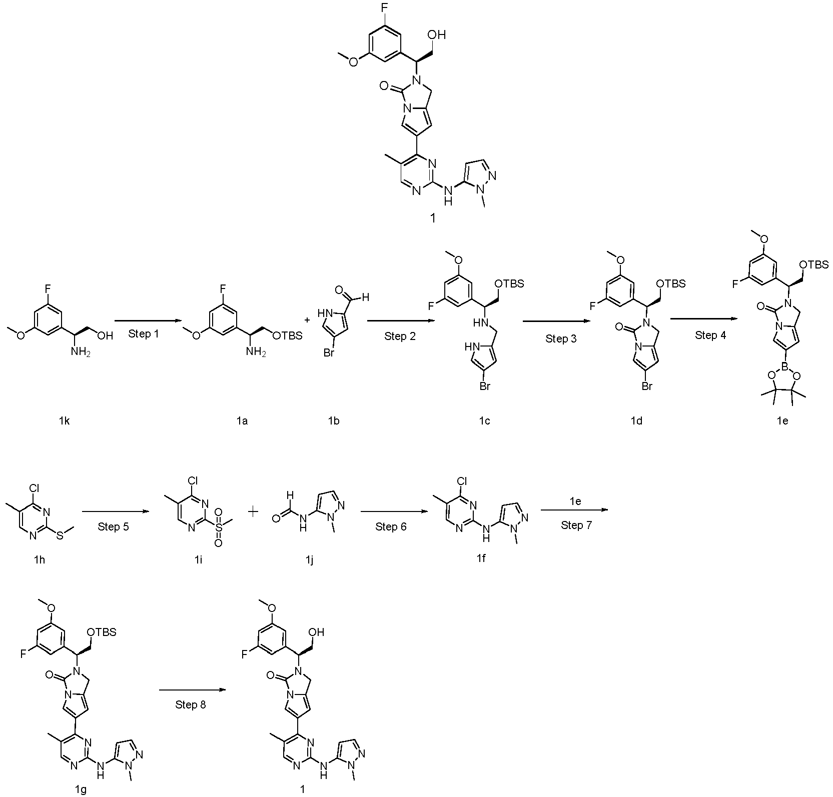

### Step 1

### (S)-2-((tert-Butyldimethylsilyl)oxy)-1-(3-fluoro-5-methoxyphenyl)ethan-1-amine 1a

(*S*)-2-Amino-2-(3-fluoro-5-methoxyphenyl)ethan-1-ol **1k** (2 g, 10.8 mmol, Shanghai Haohong Biomedical Technology Co., Ltd.) and imidazole (1.47 g, 21.6 mmol) were dissolved in 80 mL of dichloromethane, followed by the addition of *tert*-butyldimethylchlorosilane (TBSCl, 2.44 g, 16.19 mmol) in an ice bath. The reaction solution was stirred for 14 hours, followed by the addition of water, and extracted with dichloromethane (80 mL×3). The organic phases were combined, washed with saturated sodium chloride solution, dried over anhydrous sodium sulfate, and filtered. The filtrate was concentrated under reduced pressure, and the residues were purified by column chromatography with eluent system C to obtain the title compound **1a** (2.0 g), yield: 61.8%.

MS m/z (ESI): 300.2 [M+1].

### Step 2

### (S)-N-((4-Bromo-1H-pyrrol-2-yl)methyl)-2-((tert-butyldimethylsilyl)oxy)-1-(3-fluoro-5 -methoxyphenyl)ethan-1-amine 1c

(*S*)-2-((*tert*-Butyldimethylsilyl)oxy)-1-(3-fluoro-5-methoxyphenyl)ethan-1-amine **1a** (516 mg, 1.72 mmol) and compound 4-bromo-1*H*-pyrrole-2-carbaldehyde **1b** (300 mg, 1.72 mmol, Shanghai Bide Pharmatech Ltd.) were stirred and reacted for 3 hours. The reaction solution was diluted with 5 mL methanol, and cool to 0°C. Sodium borohydride (65 mg, 1.72 mmol) was added, and the reaction solution was stirred for 2 hours. Water was added, and the reaction solution was concentrated under reduced pressure. Water was added, and the reaction solution was extracted with ethyl acetate (10 mL×2). The organic phases were combined, washed with saturated sodium chloride solution, dried over anhydrous sodium sulfate, and filtered. The filtrate was concentrated under reduced pressure, and the residues were purified by column chromatography with eluent system C to obtain the title compound **1c** (500 mg), yield: 63%.

MS m/z (ESI): 457.1 [M+1].

### Step 3

### (S)-6-Bromo-2-(2-((tert-butyldimethylsilyl)oxy)-1-(3-fluoro-5-methoxyphenyl)ethyl)-1 H-pyrrolo[1,2-c]imidazol-3(2H)-one 1d

Compound **1c** (400 mg, 0.9 mmol) was dissolved in 40 mL of tetrahydrofuran, followed by the addition of *N,N'*-carbonyldiimidazole (219 mg, 1.36 mmol) in an ice bath, and the reaction solution was stirred for 0.5 hours. Sodium hydride (60%, 69 mg, 1.8 mmol) was added, and the reaction solution was stirred for 14 hours, followed by the addition of saturated ammonium chloride solution. The reaction solution was concentrated under reduced pressure, and the residues were purified by column chromatography with eluent system C to obtain the title compound **1d** (400 mg), yield: 94%.

MS m/z (ESI): 483.2 [M+1].

### Step 4

### (S)-2-(2-((tert-Butyldimethylsilyl)oxy)-1-(3-fluoro-5-methoxyphenyl)ethyl)-6-(4,4,5,5-t etramethyl-1,3,2-dioxaborolan-2-yl)-1H-pyrrolo[1,2-c]imidazol-3(2H)-one 1e

Compound **1d** (360 mg, 0.75 mmol) was dissolved in 50 mL of 1,4-dioxane under argon atmosphere, followed by the addition of 4,4,4',4',5,5,5',5'-octamethyl-2,2'-bis(1,3,2-dioxaborolane) (189 mg, 0.76 mmol), potassium acetate (219 mg, 2.23 mmol) and [1,1'-bis(diphenylphosphino)ferrocene]palladium (II) dichloride (109 g, 0.15 mmol) successively. The reaction solution was stirred for 2 hours at 90°C, cooled, and filtered through Celite. The filtrate was concentrated, and the residues were purified by column chromatography with eluent system C to obtain the title compound **1e** (100 mg), yield: 25%.

MS m/z (ESI): 531.4 [M+1].

### Step 5

### 4-Chloro-5-methyl-2-(methylsulfonyl)pyrimidine 1i

4-Chloro-5-methyl-2-(methylthio)pyrimidine **1h** (500 mg, 2.86 mmol, Shanghai Bide Pharmatech Ltd.) was dissolved in 10 mL of dichloromethane, followed by the addition of *m*-chloroperoxybenzoic acid (1.270 g, 6.3 mmol), and the reaction solution was stirred for 2 hours. The reaction solution was washed with saturated sodium thiosulfate solution and saturated sodium chloride solution, dried over anhydrous sodium sulfate, and filtered. The filtrate was concentrated under reduced pressure to obtain the crude title compound **1i** (445 mg), which was directly used in the next step without purification.

MS m/z (ESI): 207.2 [M+1].

### Step 6

### 4-Chloro-5-methyl-N-(1-methyl-1H-pyrazol-5-yl)pyrimidin-2-amine 1f

*N*-(1-Methyl-1*H*-pyrazol-5-yl)carboxamide **1j** (270 mg, 2.15 mmol, prepared by the well-known method disclosed in *"*Bioorganic and Medicinal Chemistry, 1997, 5(3), 557-567") was dissolved in *N,N*-dimethylformamide, followed by the addition of sodium hydride (60%, 250 mg, 6.5 mmol) at 0°C, and the reaction solution was stirred for 0.5 hours. Compound **1i** (445 mg, 2.15 mmol) was added, and the reaction solution was further reacted for 2 hours. 20 mL of water was added, and the reaction solution was extracted with ethyl acetate (20 mL×3). The organic phases were combined and concentrated under reduced pressure, and the resulting residues were purified by thin layer chromatography with developing solvent system C to obtain the title compound **1f** (240 mg), yield: 49.7 %.

MS m/z (ESI): 224.3 [M+1].

### Step 7

### (S)-2-(2-((tert-Butyldimethylsilyl)oxy)-1-(3-fluoro-5-methoxyphenyl)ethyl)-6-(5-methyl-2-((1-methyl-1H-pyrazol-5-yl)amino)pyrimidin-4-yl)-1H-pyrrolo[1,2-c]imidazo 1-3-one 1g

A mixture of compound **1e** (100 mg, 0.19 mmol), compound **1f** (42 mg, 0.19 mmol), [1,1'-bis(diphenylphosphino)ferrocene]palladium (II) dichloride (28 mg, 0.04 mmol) and cesium carbonate (123 mg, 0.4 mmol) was suspended in 30 mL of 1,4-dioxane and 4 mL of water under argon atmosphere. The reaction solution was heated to 80°C, and stirred for 14 hours. The reaction solution was cooled, and filtered through Celite. The filtrate was collected, and extracted with ethyl acetate (20 mL×3). The organic phases were combined, concentrated under reduced pressure, and the residues were purified by column chromatography with eluent system A to obtain the title compound **1g** (80 mg), yield: 72%.

MS m/z (ESI): 592.1 [M+1].

### Step 8

### (S)-2-(1-(3-Fluoro-5-methoxyphenyl)-2-hydroxyethyl)-6-(5-methyl-2-((1-methyl-1H-py razol-5-yl)amino)pyrimidin-4-yl)-1H-pyrrolo[1,2-c]imidazol-3(2H)-one 1

Compound **1g** (80 mg, 0.14 mmol) was dissolved in 20 mL of dichloromethane, followed by the dropwise addition of 5 mL of trifluoroacetic acid. After the addition was completed, the reaction solution was stirred for 4 hours. The pH was adjusted to 7 with saturated sodium bicarbonate solution, and the reaction solution was extracted with dichloromethane (20 mL×2). The organic phases were combined, concentrated under reduced pressure, and the residues were purified by column chromatography with eluent system A to obtain the title compound 1 (18 mg), yield: 28%.

MS m/z (ESI): 478.3 [M+1].

¹H NMR (400 MHz, CD₃OD): δ 8.21 (s, 1H), 7.71 (s, 1H), 7.43 (d, 1H), 6.79-6.68 (m, 4H), 6.32 (d, 1H), 5.23-5.22 (m, 1H), 4.64 (d, 1H), 4.39-4.35 (m, 1H), 4.18-4.08 (m, 2H), 3.81 (s, 3H), 3.75 (s, 3H), 2.40 (s, 3H).

### Example 2

### (S)-6-(5-Chloro-2-((1-methyl-1H-pyrazol-5-yl)amino)pyrimidin-4-yl)-2-(1-(3-chlorophe nyl)-2-hydroxyethyl)-1H-pyrrolo[1,2-c]imidazol-3(2H)-one 2

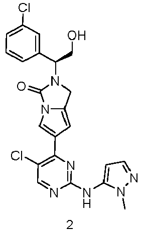

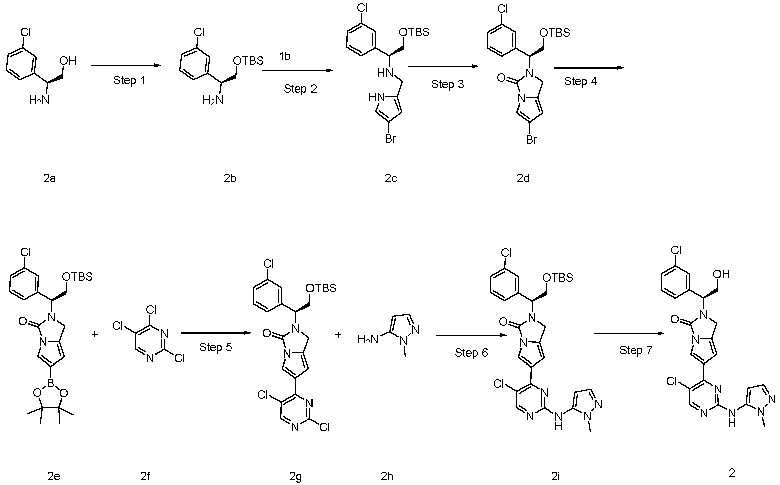

### Step 1

### (S)-2-((tert-Butyldimethylsilyl)oxy)-1-(3-chlorophenyl)ethan-1-amine 2b

(*S*)-2-Amino-2-(3-chlorophenyl)ethan-1-ol **2a** (4 g, 23.3 mmol, Shanghai Bide Pharmatech Ltd.) and imidazole (3.2 g, 46.6 mmol) were dissolved in 80 mL of dichloromethane, followed by the addition of t*ert*-butyldimethylchlorosilane (5.2 g, 35 mmol) in an ice bath, and the reaction solution was stirred for 14 hours. Water was added, and the reaction solution was extracted with dichloromethane (80 mL×3). The organic phases were combined, washed with saturated sodium chloride solution, dried over anhydrous sodium sulfate, and filtered. The filtrate was concentrated under reduced pressure, and the residues were purified by column chromatography with eluent system C to obtain the title compound **2b** (6.5 g), yield: 97%.

MS m/z (ESI): 286.1 [M+1].

### Step 2

### (S)-N-((4-Bromo-1H-pyrrol-2-yl)methyl)-2-((tert-butyldimethylsilyl)oxy)-1-(3-chlorop henyl)ethan-1-amine 2c

Compound **1b** (2.37 g, 13.62 mmol) and compound **2b** (3.9 g, 13.64 mmol) were stirred and reacted for 3 hours. The reaction solution was diluted with 100 mL of methanol, cooled to 0°C, followed by the addition of sodium borohydride (516 mg, 13.64 mmol), and stirred for 2 hours. Water was added, and the reaction solution was concentrated under reduced pressure. Water was added, and the reaction solution was extracted with ethyl acetate (40 mL×3). The organic phases were combineed, washed with saturated sodium chloride solution, dried over anhydrous sodium sulfate, and filtered. The filtrate was concentrated under reduced pressure, and the residues were purified by column chromatography with eluent system C to obtain the title compound **2c** (4.8 g), yield: 79%.

MS m/z (ESI): 444.2 [M+1].

### Step 3

### (S)-6-Bromo-2-(2-((tert-butyldimethylsilyl)oxy)-1-(3-chlorophenyl)ethyl)-1H-pyrrolo[1 ,2-c]imidazol-3(2H)-one 2d

Compound **2c** (4.8 g, 10.81 mmol) was dissolved in 100 mL of tetrahydrofuran, followed by the addition of *N,N'*-carbonyldiimidazole (2.45 g, 15.11 mmol) in an ice bath, and the reaction solution was stirred for 0.5 hours. Sodium hydride (60%, 621 mg, 16.22 µmol) was added, and the reaction solution was stirred for 14 hours at room temperature, followed by the addition of saturated ammonium chloride solution. The reaction solution was concentrated under reduced pressure, and the residues were purified by column chromatography with eluent system C to obtain the title compound **2d** (4.0 g), yield: 78%.

MS m/z (ESI): 469.1 [M+1].

### Step 4

### (S)-2-(2-((tert-Butyldimethylsilyl)oxy)-1-(3-chlorophenyl)ethyl)-6-(4,4,5,5-tetramethyl-1,3,2-dioxaborolan-2-yl)-1H-pyrrolo[1,2-c]imidazol-3(2H)-one 2e

Under argon atmosphere, compound **2d** (4.0 g, 8.51 mmol) was dissolved in 50 mL of 1,4-dioxane, followed by the addition of 4,4,4',4',5,5,5',5'-octamethyl-2,2'-bis(1,3,2-dioxaborolane) (3.24 g, 12.76 mmol), potassium acetate (3.34 g, 34.04 mmol) and [1,1'-bis(diphenylphosphino)ferrocene]palladium (II) dichloride (1.24 g, 1.70 mmol) successively. The reaction solution was stirred for 2 hours at 90°C, cooled, and filtered through Celite. The filtrate was concentrated, and the residues were purified by column chromatography with eluent system C to obtain the title compound **2e** (2.0 g), yield: 45%.

MS m/z (ESI): 517.2 [M+1].

### Step 5

### (S)-2-(2-((tert-Butyldimethylsilyl)oxy)-1-(3-chlorophenyl)ethyl)-6-(2,5-dichloropyrimid in-4-yl)-1H-pyrrolo[1,2-c]imidazol-3(2H)-one 2g

A mixture of compound **2e** (430 mg, 0.83 mmol), 2,4,5-trichloropyrimidine **2f** (183 mg, 0.99 mmol, Shanghai Bide Pharmatech Ltd.), [1,1'-bis(diphenylphosphino)ferrocene]palladium (II) dichloride (60 mg, 0.08 mmol) and sodium carbonate (175 mg, 1.65 mmol) was suspended in 2 mL of 1,4-dioxane and 1 mL of water under argon atmosphere. The reaction system was purged with argon, and stirred for 1.5 hours in microwave reactor at 85°C. The reaction solution was cooled, and filtered through Celite. The filtrate was collected, and extracted with ethyl acetate (10 mL×3). The organic phases were combined and concentrated, and the residues were purified by thin layer chromatography with developing solvent system C to obtain the title compound **2g** (200 mg), yield: 44%.

MS m/z (ESI): 537.1 [M+1].

### Step 6

### (S)-2-(2-((tert-Butyldimethylsilyl)oxy)-1-(3-chlorophenyl)ethyl)-6-(5-chloro-2-((1-meth yl-1H-pyrazol-5-yl)amino)pyrimidin-4-yl)-1H-pyrrolo[1,2-c]imidazol-3(2H)-one 2i

Compound **2g** (100 mg, 0.18 mmol) was dissolved in 1 mL of 1,4-dioxane under an argon atmosphere, followed by the addition of tris(dibenzylideneacetone)dipalladium (25 mg, 27.30 µmol), 4,5-bis(diphenylphosphino)-9,9-dimethylxanthene (32 mg, 55.30 µmol), cesium carbonate (121 mg, 0.37 mmol) and 1-methyl-1*H*-pyrazol-5-amine **2h** (36 mg, 0.37 mmol, Shanghai Bide Pharmatech Ltd.), and the reaction solution was stirred for 1 hour at 100°C in microwave reactor. The reaction solution was cool and filtered through Celite. The filtrate was concentrated, and the residues were purified by thin layer chromatography with developing solvent system A to obtain the title compound **2i** (50 mg), yield: 44%.

MS m/z (ESI): 597.9 [M+1].

### Step 7

### (S)-6-(5-Chloro-2-((1-methyl-1H-pyrazol-5-yl)amino)pyrimidin-4-yl)-2-(1-(3-chlorophe nyl)-2-hydroxyethyl)-1H-pyrrolo[1,2-c]imidazol-3(2H)-one 2

Compound **2i** (50 mg, 83.52 µmol) was dissolved in 3 mL of dichloromethane, followed by the addition of 1 mL of trifluoroacetic acid dropwise. After the addition was completed, the reaction solution was stirred for 3 hours. The pH was adjusted to 7 with saturated sodium bicarbonate solution, and the reaction solution was extracted with dichloromethane (10 mL×2). The organic phases were combined and concentrated, and the residues were purified by column chromatography with eluent system A to obtain the title compound 2 (16 mg), yield: 39%.

MS m/z (ESI): 484.0 [M+1].

¹H NMR (400 MHz, CD₃OD): δ 8.35 (s, 1H), 8.08 (s, 1H), 7.49-7.42 (m, 2H), 7.36 (m, 3H), 6.83 (s, 1H), 6.36 (d, 1H), 5.24 (dd, 1H), 4.64 (d, 1H), 4.34 (d, 1H), 4.25-4.15 (m, 1H), 4.12-4.04 (m, 1H), 3.76 (s, 3H).

### Example 3

### (S)-2-(1-(3-Chlorophenyl)-2-hydroxyethyl)-6-(5-methyl-2-((1-methyl-1H-pyrazol-5-yl) amino)pyrimidin-4-yl)-1H-pyrrolo[1,2-c]imidazol-3(2H)-one 3

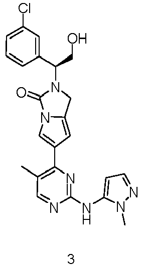

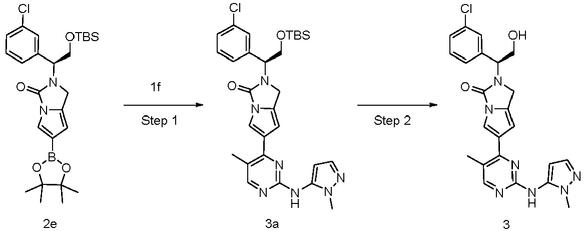

### Step 1

### (S)-2-(2-((tert-Butyldimethylsilyl)oxy)-1-(3-chlorophenyl)ethyl)-6-(5-methyl-2-((1-met hyl-1H-pyrazol-5-yl)amino)pyrimidin-4-yl)-1H-pyrrolo[1,2-c]imidazol-3(2H)-one 3a

A mixture of compound **1f** (800 mg, 3.57 mmol), compound **2e** (2.03 g, 3.93 mmol), [1,1'-bis(diphenylphosphino)ferrocene]palladium (II) dichloride (392 mg, 0.54 mmol) and cesium carbonate (2.33 g, 7.15 mmol) was suspended in 30 mL of 1,4-dioxane and 4 mL of water under argon atmosphere. The reaction solution was heated to 80°C, and stirred for 14 hours. The reaction solution was cooled, and filtered through Celite. The filtrate was collected, and extracted with ethyl acetate (40 mL×3). The organic phases were combined and concentrated, and the residues were purified by column chromatography with eluent system A to obtain the title compound **3a** (1.2 g), yield: 58%.

MS m/z (ESI): 578.3 [M+1].

### Step 2

### (S)-2-(1-(3-Chlorophenyl)-2-hydroxyethyl)-6-(5-methyl-2-((1-methyl-1H-pyrazol-5-yl)amino)pyrimidin-4-yl)-1H-pyrrolo[1,2-c]imidazol-3(2H)-one 3

Compound **3a** (1.2 g, 2.07 mmol) was dissolved in 20 mL of dichloromethane, followed by the addition of 5 mL of trifluoroacetic acid dropwise. After the addition was completed, the reaction solution was stirred for 4 hours. The pH was adjusted to 7 with saturated sodium bicarbonate solution, and the reaction solution was extracted with dichloromethane (20 mL×2). The organic phases were combined and concentrated, and the residues were purified by column chromatography with eluent system A to obtain the title compound **3** (600 mg), yield: 62%.

MS m/z (ESI): 464.2 [M+1].

¹H NMR (400 MHz, CD₃OD): δ 8.21 (s, 1H), 7.71 (s, 1H), 7.46 (s, 2H), 7.42-7.30 (m, 3H), 6.75 (s, 1H), 6.36 (s, 1H), 5.26 (dd, 1H), 4.66 (d, 1H), 4.36 (d, 1H), 4.26-4.16 (m, 1H), 4.14-4.02 (m, 1H), 3.76 (s, 3H), 2.39 (s, 3H).

### Example 4

### (S)-6-(5-Chloro-2-((tetrahydro-2H-pyran-4-yl)amino)pyrimidin-4-yl)-2-(1-(3-chlorophe nyl)-2-hydroxyethyl)-1H-pyrrolo[1,2-c]imidazol-3(2H)-one 4

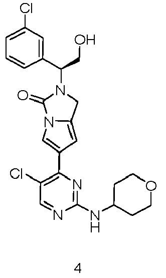

The synthetic route of Example 2 was applied, with the replacement of starting compound **2h** in Step 6 with compound tetrahydro-2*H*-pyran-4-amine (Shanghai Bide Pharmatech Ltd.), to obtain compound **5** (12 mg).

MS m/z (ESI): 488.0 [M+1].

¹H NMR (400 MHz, CDCl₃): δ 8.24 (s, 1H), 8.13 (s, 1H), 7.44-7.33 (m, 3H), 7.27 (s, 1 H), 6.82 (s, 1H), 5.17 (dd, 1H), 5.10 (d, 1H), 4.49 (d, 1H), 4.38-4.18 (m, 3H), 4.04 (d, 1H), 4.02 (d, 1H), 3.66-3.50 (m, 2H), 2.09 (d, 2H), 1.3 -1.28 (m, 2H).

### Example 5

### 6-(5-Chloro-2-(((S)-1-hydroxypropan-2-yl)amin)pyrimidin-4-yl)-2-((S)-1-(3-chlorophe nyl)-2-hydroxyethyl)-1H-pyrrolo[1,2-c]imidazol-3(2H)-one 5

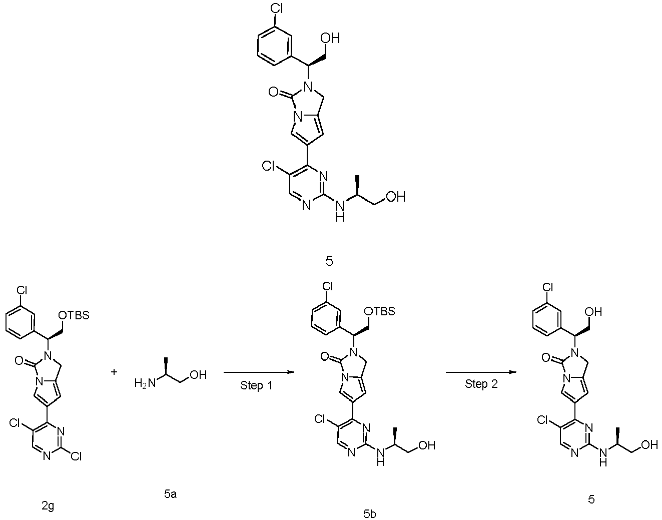

### Step 1

### 2-((S)-2-((tert-Butyldimethylsilyl)oxy)-1-(3-chlorophenyl)ethyl)-6-(5-chloro-2-(((S)-1-h ydroxypropan-2-yl)amino)pyrimidin-4-yl)-1,2-dihydro-3H-pyrrolo[1,2-c]imidazol-3-on e 5b

Compound **2g** (30 mg, 55.77 µmol) and (*S*)-2-aminopropan-1-ol **5a** (21 mg, 279.59 µmol, Shanghai Bide Pharmatech Ltd.) were dissolved in 1 mL of tetrahydrofuran, and the reaction solution was stirred for 1 hour at 110°C in microwave reactor. The reaction solution was concentrated under reduced pressure to obtain the crude title compound **5b** (32 mg), which was directly used in the next step.

MS m/z (ESI): 576.2 [M+1].

### Step 2

### 6-(5-Chloro-2-(((S)-1-hydroxypropan-2-yl)amino)pyrimidin-4-yl)-2-((S)-1-(3-chlor ophenyl)-2-hydroxyethyl)-1H-pyrrolo[1,2-c]imidazol-3(2H)-one 5

Compound **5b** (30 mg, 52.03 µmol) was dissolved in 3 mL of dichloromethane, followed by the addition of 1 mL of trifluoroacetic acid dropwise. After the addition was completed, the reaction solution was stirred for 3 hours. The pH was adjusted to 7 with saturated sodium bicarbonate solution, and the reaction solution was extracted with dichloromethane (5 mL×2). The organic phases were combined and concentrated, and the residues were purified by column chromatography with eluent system A to obtain the title compound **5** (10.8 mg), yield: 44%.

MS m/z (ESI): 462.0 [M+1].

¹H NMR (400 MHz, CD₃OD): δ 8.24 (s, 1H), 8.15 (s, 1H), 7.46 (s, 1H), 7.40-7.34 (m, 3H), 6.93 (s, 1H), 5.26 (dd, 1H), 4.67 (d, 1H), 4.37 (d, 1H), 4.24-4.06 (m, 3H), 3.67-3.56 (m, 2H), 1.27 (d, 3H).

### Example 6

### (S)-2-(1-(3-Chloro-4-fluorophenyl)-2-hydroxyethyl)-6-(5-methyl-2-((1-methyl-1H-pyra zol-5-yl)amino)pyrimidin-4-yl)-1H-pyrrolo[1,2-c]imidazol-3(2H)-one 6

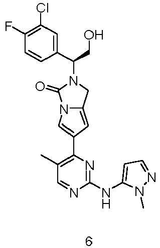

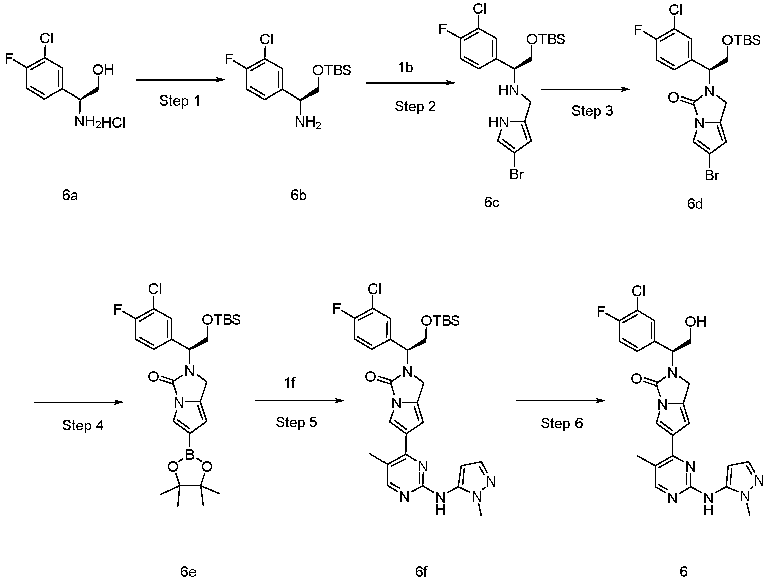

### Step 1

### (S)-2-((tert-Butyldimethylsilyl)oxy)-1-(3-chloro-4-fluorophenyl)ethamine 6b

(*S*)-2-Amino-2-(3-chloro-4-fluorophenyl)ethan-1-ol hydrochloride **6a** (250 mg, 1.1 mmol, Shanghai Bide Pharmatech Ltd.) was dissolved in 10 mL of dichloromethane, followed by the addition of imidazole (225.84 mg, 3.3174 mmol). The reaction solution was cooled to 0°C, followed by the addition of *tert*-butyldimethylchlorosilane (250 mg, 1.7 mmol), and stirred for 14 hours. 20 mL of water was added, and the reaction solution was extracted with dichloromethane, dried over anhydrous sodium sulfate, and filtered. The filtrate was concentrated under reduced pressure, and the residues were purified by column chromatography with eluent system C to obtain the title compound **6b** (290 mg), yield: 86%.

MS m/z (ESI): 304.1 [M+1].

### Step 2

### (S)-N-((4-Bromo-1H-pyrrol-2-yl)methyl)-2-((tert-butyldimethylsilyl)oxy)-1-(3-chloro-4 -fluorophenyl)ethamine 6c

Compound **6b** (290 mg, 0.95 mmol) and compound **1b** (166.05 mg, 0.95 mmol) were stirred and reacted for 3 hours, followed by the addition of 5 mL of methanol. The reaction solution was cooled to 0°C, followed by the addition of sodium borohydride (36 mg, 0.95 mmol), and stirred for 2 hours. Water was added, and the reaction solution was concentrated under reduced pressure. Water was added, and the reaction solution was extracted with ethyl acetate. The organic phases were combined, washed with saturated sodium chloride solution, dried over anhydrous sodium sulfate, and filtered. The filtrate was concentrated under reduced pressure, and the residues were purified by column chromatography with eluent system C to obtain the title compound **6c** (300 mg), yield: 68%.

MS m/z (ESI): 459.1 [M-1].

### Step 3

### (S)-6-Bromo-2-(2-((tert-butyldimethylsilyl)oxy)-1-(3-chloro-4-fluorophenyl)ethyl)-1H-pyrrolo[1,2-c]-3(2H)-one 6d

Compound **6c** (300 mg, 0.65 mmol) was dissolved in 40 mL of tetrahydrofuran, followed by the addition of *N,N'*-carbonyldiimidazole (147 mg, 0.91 mmol) in ice bath, and the reaction solution was stirred for 0.5 hours. Sodium hydride (60%, 37 mg, 0.97 mmol) was added, and the reaction solution was stirred for 14 hours at room temperature, followed by the addition of saturated ammonium chloride solution. The reaction solution was concentrated under reduced pressure, and the residues were purified by column chromatography with eluent system C to obtain the title compound **6d** (300 mg), yield: 94%.

MS m/z (ESI): 487.2 [M+1].

### Step 4

### (S)-2-(2-((tert-Butyldimethylsilyl)oxy)-1-(3-chloro-4-fluorophenyl)ethyl)-6-(4,4,5,5-tetr amethyl-1,3,2-dioxaborolan-2-yl)-1H-pyrrolo[1,2-c]imidazol-3(2H)-one 6e

Compound **6d** (300 mg, 0.61 mmol) was dissolved in 50 mL of dioxane under argon atmosphere, followed by the addition of 4,4,4',4',5,5,5',5'-octamethyl-2,2'-bis(1,3,2-dioxaborolane) (254 mg, 0.92 mmol), potassium acetate (181 mg, 1.84 mmol) and [1,1'-bis(diphenylphosphino)ferrocene]palladium (II) dichloride (90 mg, 1.23mmol) successively. The reaction solution was stirred for 2 hours at 90°C, cooled, and filtered through Celite. The filtrate was concentrated, and the residues were purified by column chromatography with eluent system C to obtain the title compound **6e** (120 mg), yield: 36%.

MS m/z (ESI): 534.1 [M+1].

### Step 5

### (S)-2-(2-((tert-Butyldimethylsilyl)oxy)-1-(3-chloro-4-fluorophenyl)ethyl)-6-(5-met hyl-2-((1-methyl-1H-pyrazol-5-yl)amino)pyrimidin-4-yl)-1,2-dihydro-3H-pyrrolo[1,2-c ]imidazol-3-one 6f

A mixture of compound **1f** (50 mg, 0.22 mmol), compound **6e** (120 mg, 0.22 mmol), [1,1'-bis(diphenylphosphino)ferrocene]palladium (II) dichloride (33 mg, 0.04 mmol) and cesium carbonate (146 mg, 0.45 mmol) was suspended in 30 mL of 1,4-dioxane and 6 mL of water under an argon atmosphere, and the reaction solution was heated to 80°C, and stirred for 14 hours. The reaction solution was cooled, and filtered through Celite. The filtrate was collected, and extracted with ethyl acetate (15 mL×2). The organic phases were combined and concentrated under reduced pressure, and the residues were purified by column chromatography with eluent system A to obtain the title compound **6f** (100 mg), yield: 74%.

MS m/z (ESI): 596.1 [M+1].

### Step 6

### (S)-2-(1-(3-Chloro-4-fluorophenyl)-2-hydroxyethyl)-6-(5-methyl-2-((1-methyl-1H-pyrazol-5-yl)amino)pyrimidin-4-yl)-1H-pyrrolo[1,2-c]imidazol-3(2H)-one 6

Compound **6f** (100 mg, 0.17 mmol) was dissolved in 3 mL of dichloromethane, followed by the addition of 1 mL of trifluoroacetic acid dropwise. After the addition was completed, the reaction solution was stirred for 4 hours. The pH was adjusted to 7 with saturated sodium bicarbonate solution, and the reaction solution was extracted with dichloromethane (15 mL×2). The organic phases were combined and concentrated under reduced pressure, and the residues were purified by thin layer chromatography with developing solvent system A to obtain the title compound **6** (15 mg), yield: 18%.

MS m/z (ESI): 482.2 [M+1].

¹H NMR (400 MHz, CD₃OD): δ 8.19 (s, 1H), 7.55 (s, 1H), 7.52-7.44 (m, 2H), 7.41 (d, 1H), 7.22-7.13 (m, 1H), 6.54 (s, 1H), 6.14 (d, 1H), 5.14 (dd, 1H), 4.42 (d, 1H), 4.32 (dd, 1H), 4.23 - 4.12 (m, 2H), 3.78 (s, 3H), 2.33 (s, 3H).

### Example 7

### 6-(5-Chloro-2-((3-fluoro-4-hydroxycyclopentyl)amino)pyrimidin-4-yl)-2-((S)-1-(3-chlo rophenyl)-2-hydroxyethyl)-1H-pyrrolo[1,2-c]imidazol-3(2H)-one 7

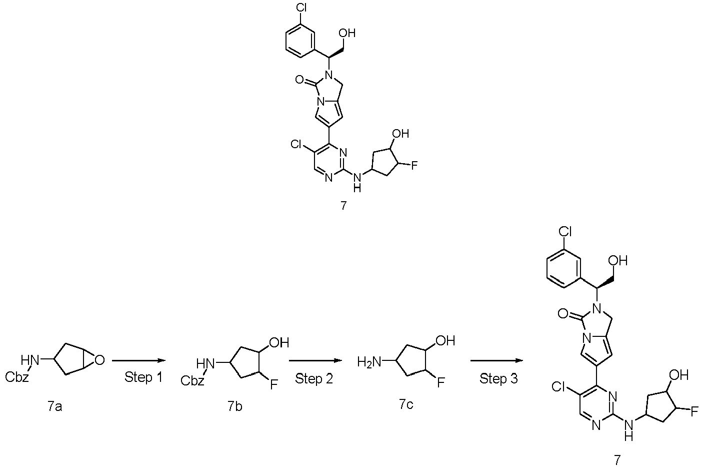

### Step 1

### Benzyl (3-fluoro-4-hydroxycyclopentyl)carbamate 7b

Benzyl (6-oxabicyclo[3.1.0]hexan-3-yl)carbamate **7a** (1 g, 4.28 mmol, prepared by the well-known method disclosed in *"*Tetrahedron, 56 (2000) 9633-9640") and hydrogen fluoride pyridine complex (1.06 g, 6.41 mmol, purity 60%) were dissolved in 1,2-dichloroethane (5 mL), and the reaction solution was stirred for 4 hours. The reaction solution was washed with saturated aqueous sodium bicarbonate solution and saturated sodium chloride, dried over anhydrous sodium sulfate, and filtered. The filtrate was concentrated under reduced pressure, and the residues were purified by thin layer chromatography with developing solvent system C to obtain the compound **7b** (500 mg), yield: 46%.

MS m/z (ESI): 253.6 [M+1].

### Step 2

### 4-Amino-2-fluorocyclopentanol 7c

Compound **7b** (150 mg, 592.25 µmol) and 10% palladium-carbon hydrogenation catalyst (wet) (30 mg, 281.90 µmol) were dissolved in 5 mL of methanol under a hydrogen atmosphere. The reaction solution was stirred for 16 hours and filtered. The filtrate was concentrated under reduced pressure to obtain the crude compound **7c** (150 mg), which was directly used in the next reaction.

### Step 3

### 6-(5-Chloro-2-((3-fluoro-4-hydroxycyclopentyl)amino)pyrimidin-4-yl)-2-((S)-1-(3 -chlorophenyl)-2-hydroxyethyl)-1H-pyrrolo[1,2-c]imidazol-3(2H)-one 7

The synthetic route in Example 2 was applied with the replacement of the starting compound **2h** in Step 6 with compound **7c,** to obtain compound **7** (5 mg).

MS m/z (ESI): 506.1 [M+1].

¹H NMR (400 MHz, CDCl₃): δ 8.18 (s, 1H), 8.07 (d, 1H), 7.36 (d, 3H), 7.27 (d, 1H), 6.79 (s, 1H), 5.62 (d, 1H), 5.19 (td, 1H), 4.96 (dd, 1H), 4.50 (d, 2H), 4.38-4.15 (m, 4H), 2.60-2.48 (m, 1H), 2.46-2.29 (m, 2H), 1.85 (d, 2H).

### Example 8

### 6-(5-Chloro-2-(((R)-1-hydroxypropan-2-yl)amino)pyrimidin-4-yl)-2-((S)-1-(3-chloroph enyl)-2-hydroxyethyl)-1,2-dihydro-3H-pyrrolo[1,2-c]imidazol-3-one 8

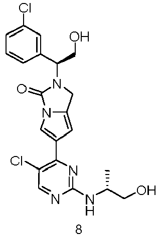

The synthetic route in Example 5 was applied with the replacement of the starting compound **5a** in Step 1 with (*R*)-2-aminopropane-1-ol, to obtain compound **8** (17 mg).

MS m/z (ESI): 462.0 [M+1].

¹H NMR (400 MHz, CD₃OD): δ 8.23 (s, 1H), 8.15 (s, 1H), 7.45 (s, 1H), 7.42-7.30 (m, 3H), 6.92 (s, 1H), 5.25 (dd, 1H), 4.67 (d, 1H), 4.36 (d, 1H), 4.24 - 4.04 (m, 3H), 3.62 (t, 2H), 1.27 (d, 3H).

### Example 9

### (S)-2-(1-(3-Chlorophenyl)-2-hydroxyethyl)-6-(5-methyl-2-((1-methyl-1H-pyrazol-5-yl) amino)pyrimidin-4-yl)-3,4-dihydropyrrolo[1,2-c]pyrimidin-1(2H)-one 9

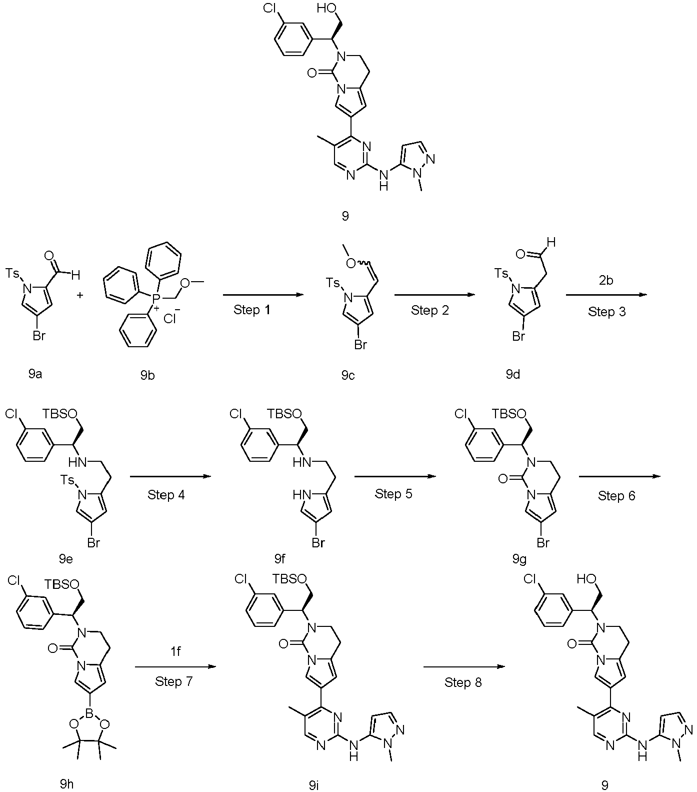

### Step 1

### 4-Bromo-2-(2-methoxyvinyl)-1-tosyl-1H-pyrrole 9c

(Methoxymethyl)triphenylphosphonium chloride **9b** (8.36 g, 24.37 mmol) was dissolved in 150 mL of tetrahydrofuran, and the resulting solution was cooled to 0°C. Potassium *tert*-butoxide (2.74 g, 24.37 mmol) was added, and the reaction solution was stirred for 20 minutes. 4-Bromo-1-tosyl-1*H*-pyrrole-2-carbaldehyde **9a** (4.0 g, 612.19 mmol, prepared by the well-known method disclosed in *"*Journal of Porphyrins and Phthalocyanines, 2009, 13(10), 1098-1110"), and the reaction solution was stirred overnight, followed by the addition of water. The reaction solution was concentrated under reduced pressure, and the residues were purified by column chromatography with eluent system C to obtain the title compound **9c** (4.0 g), yield: 92%.

MS m/z (ESI): 356.0 [M+1].

### Step 2

### 2-(4-Bromo-1-tosyl-1H-pyrrol-2-yl)acetaldehyde 9d

Compound **9c** (3 g, 8.42 mmol) was dissolved in 10 mL tetrahydrofuran, followed by the addition of 10 mL concentrated hydrochloric acid, and the reaction solution was stirred for 3 hours. The pH was adjusted to 7 with saturated sodium bicarbonate. The reaction solution was extracted with ethyl acetate (50 mL×2), and the organic phase was concentrated to obtain the title crude compound **9d** (2.88 g), which was directly used in the next step without purification.

MS m/z (ESI): 342.2 [M+1].

### Step 3

### (S)-N-(2-(4-Bromo-1-tosyl-1H-pyrrol-2-yl)ethyl)-2-((tert-butyldimethylsilyl)oxy)-1-(3-chlorophenyl)ethan-1-amine 9e

The crude compound **9d** (2.88 g, 8.42 mmol) and compound **2b** (2 g, 6.99 mmol) were dissolved in 20 mL methanol, and the reaction solution was stirred for 1 hour. Sodium borohydride (396 mg, 10.48 mmol) was added, and the reaction solution was stirred for 14 hours at room temperature, and quenched with water. The reaction solution was concentrated under reduced pressure, and the residues were purified by column chromatography with eluent system C to obtain the title compound **9e** (2.2 g), yield: 51.4%.

MS m/z (ESI): 611.1 [M+1].

### Step 4

### (S)-N-(2-(4-Bromo-1H-pyrrol-2-yl)ethyl)-2-((tert-butyldimethylsilyl)oxy)-1-(3-chlorop henyl)ethan-1-amine 9f

Compound **9e** (500 mg, 0.82 mmol) was dissolved in 5 mL of tetrahydrofuran, followed by the addition of a solution of sodium methoxide in methanol (441 mg, 8.16 mmol, 50%) at 0°C, and the reaction solution was stirred for 4 hours. The pH was adjusted to 7 with 2 N hydrochloric acid, and the reaction solution was extracted with ethyl acetate (10 mL×2). The organic phase was concentrated under reduced pressure, and the residues were purified by thin layer chromatography with developing solvent system C to obtain the compound **9f** (280 mg), yield: 74.8%.

MS m/z (ESI): 459.1 [M+1].

### Step 5

### (S)-6-Bromo-2-(2-((tert-butyldimethylsilyl)oxy)-1-(3-chlorophenyl)ethyl)-3,4-dihydrop yrrolo[1,2-c]pyrimidin-1(2H)-one 9g

Compound **9f** (280 mg, 0.61 mmol) was dissolved in 10 mL of tetrahydrofuran, followed by the addition of *N,N'*-carbonyldiimidazole (198 mg, 1.22 mmol), and the reaction solution was stirred for 30 minutes. Sodium hydride (60%, 47 mg, 1.23 mmol) was added, and the reaction solution was stirred for 14 hours, followed by the addition of water. The reaction solution was concentrated under reduced pressure, and the residues were purified by thin layer chromatography with developing solvent system C to obtain the compound **9g** (220 mg), yield: 74%.

MS m/z (ESI): 483.1[M+1],

### Step 6

### (S)-2-(2-((tert-Butyldimethylsilyl)oxy)-1-(3-chlorophenyl)ethyl)-6-(4,4,5 ,5-tetramethyl-1,3,2-dioxaborolan-2-yl)-3,4-dihydropyrrolo[1,2-c]pyrimidin-1 (2H)-one 9h

Compound **9g** (220 mg, 0.45 mmol) was dissolved in 3 mL of 1,4-dioxane under argon atmosphere, followed by the addition of 4,4,4',4',5,5,5',5'-octamethyl-2,2'-bis(1,3,2-dioxaborolane) (173 mg, 0.68 mmol), potassium acetate (178 mg, 1.81 mmol) and [1,1'-bis(diphenylphosphino)ferrocene]palladium (II) dichloride (33 mg, 0.05 mmol) successively. The reaction solution was stirred for 4 hours at 90°C, cooled, and filtered through Celite. The filtrate was concentrated to obtain the title crude compound **9h** (240 mg), which was directly used in the next step without purification.

MS m/z (ESI): 530.9 [M+1].

### Step 7

### (S)-2-(2-((tert-Butyldimethylsilyl)oxy)-1-(3-chlorophenyl)ethyl)-6-(5-methyl-2-((1-met hyl-1H-pyrazol-5-yl)amino)pyrimidin-4-yl)-3,4-dihydropyrrolo[1,2-c]pyrimidin-1(2H)-one 9i

Compound **1f** (100 mg, 0.45 mmol) was dissolved in 6 mL of dioxane under an argon atmosphere, followed by the addition of **9h** (241 mg, 0.45 mmol), cesium carbonate (291 mg, 0.89 mmol) and [1,1'-bis(diphenylphosphino)ferrocene]palladium (II) dichloride (65 mg, 0.088 mmol) successively. The reaction solution was stirred for 1.5 hours in microwave reactor at 85°C, cooled, and filtered through Celite. The filtrate was concentrated, and the residues were purified by column chromatography with eluent system A to obtain the title compound **9i** (100 mg), yield: 37.7%.

MS m/z (ESI): 592.2[M+1],

### Step 8

### (S)-2-(1-(3-Chlorophenyl)-2-hydroxyethyl)-6-(5-methyl-2-((1-methyl-1H-pyrazol-5-yl) amino)pyrimidin-4-yl)-3 ,4-dihydropyrrolo[1,2-c]pyrimidin-1(2H)-one 9

Compound **9i** (100 mg, 0.17 mmol) was dissolved in 3 mL of dichloromethane, followed by the addition of 1 mL of trifluoroacetic acid dropwise. After the addition was completed, the reaction solution was stirred for 4 hours. The pH was adjusted to 7 with saturated sodium bicarbonate solution, and the reaction solution was extracted with dichloromethane (50 mL×2). The organic phases were combined and concentrated, and the residues were purified by column chromatography with eluent system A to obtain the title compound **9** (22 mg), yield: 27%.

MS m/z (ESI): 478.2 [M+1].

¹H NMR (400 MHz, CD₃OD): δ 8.18 (s, 1H), 7.88 (s, 1H), 7.46-7.43 (m, 2H), 7.39-7.34 (m, 3H), 6.62 (s, 1H), 6.31 (s, 1H), 5.72-5.69 (m, 1H), 4.15-4.14 (d, 2H), 3.74 (s, 3H), 3.66-3.60 (m, 1H), 3.35 (m, 1H), 3.04-3.00 (m, 1H), 2.99-2.88 (m, 1H), 2.38 (s, 3H).

### Example 10

### (S)-2-(1-(3-Chlorophenyl)-2-hydroxyethyl)-6-(2-((1-methyl-1H-pyrazol-5-yl)amino)pyr imidin-4-yl)-1,2-dihydro-3H-pyrrolo[1,2-c]imidazol-3-one 10

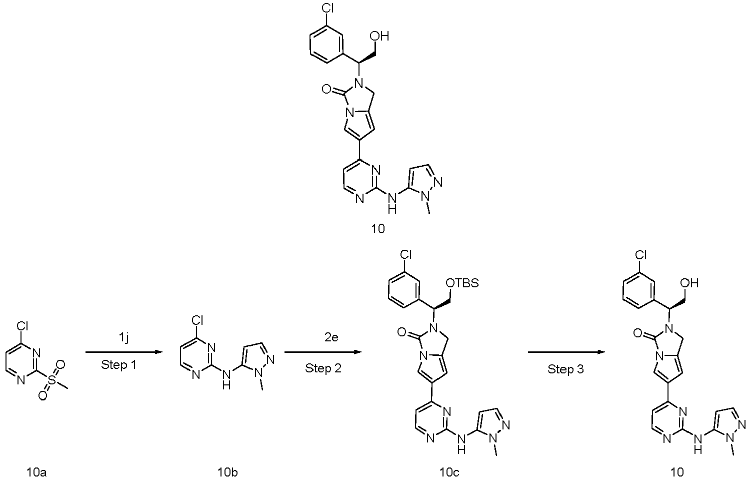

### Step 1

### 4-Chloro-N-(1-methyl-1H-pyrazol-5-yl)pyrimidin-2-amine 10b

*N*-(1-Methyl-1*H*-pyrazol-5-yl)formamide **1j** (324.82 mg, 2.60 mmol) was dissolved in 15 mL of *N,N*-dimethylformamide, followed by the addition of sodium hydride (60%, 311.47 mg, 7.79 mmol) at 0°C, and the reaction solution was stirred for 0.5 hours. Compound **10a** (500 mg, 2.60 mmol) was added, and the reaction solution was further reacted for 2 hours. 20 mL of water was added, and the reaction solution was extracted with ethyl acetate (20 mL×3). The organic phases were combined and concentrated under reduced pressure, and the residues were purified by thin layer chromatography with developing solvent system C to obtain the compound **10b** (270 mg), yield: 49.6%.

MS m/z (ESI): 210.3 [M+1].

### Step 2

### (S)-2-(2-((tert-Butyldimethylsilyl)oxy)-1-(3-chlorophenyl)ethyl)-6-(2-((1-methyl-1H-py razol-5-yl)amino)pyrimidin-4-yl)-1,2-dihydro-3H-pyrrolo[1,2-c]imidazol-3(2H)-one 10c

A mixture of compound **2e** (98.6 mg, 0.19 mmol), 4-chloro-*N*-(1-methyl-1*H*-pyrazol-5-yl)pyrimidin-2-amine **10b**, [1,1'-bis(diphenylphosphino)ferrocene]palladium (II) dichloride (28 mg, 0.02 mmol) and cesium carbonate (124 mg, 0.2 mmol) was suspended in 20 mL of 1,4-dioxane and 4 mL of water under argon atmosphere. The reaction solution was heated to 80°C, and stirred for 14 hours. The reaction solution was cooled, and filtered through Celite. The filtrate was collected, and extracted with ethyl acetate (20 mL×3). The organic phases were combined, concentrated under reduced pressure, and the residues were purified by column chromatography with eluent system A to obtain the title compound **10c** (100 mg), yield: 92%.

MS m/z (ESI): 564.3 [M+1].

### Step 3

### (S)-2-(1-(3-Chlorophenyl)-2-hydroxyethyl)-6-(2-((1-methyl-1H-pyrazol-5-yl)amino)pyr imidin-4-yl)-1,2-dihydro-3H-pyrrolo[1,2-c]imidazol-3-one 10

Compound **10c** (100 mg, 0.17 mmol) was dissolved in 20 mL of dichloromethane, followed by the addition of 1 mL of trifluoroacetic acid dropwise. After the addition was completed, the reaction solution was stirred for 4 hours. The pH was adjusted to 7 with saturated sodium bicarbonate solution, and the reaction solution was extracted with dichloromethane (20 mL×2). The organic phases were combined, concentrated under reduced pressure, and the residues were purified by column chromatography with eluent system A to obtain the title compound **10** (15 mg), yield: 18%.

MS m/z (ESI): 450.1 [M+1].

1H NMR (400 MHz, CDCl₃): δ 8.33 (d, 1H), 7.72 (s, 1H), 7.48 (d, 1H), 7.41-7.33 (m, 3H), 7.28-7.24 (m, 1H), 7.18 (s, 1H), 6.92 (d, 1H), 6.51 (s, 1H), 6.32 (d, 1H), 5.17 (dd, 1H), 4.46 (d, 1H), 4.32 (dd, 1H), 4.27-4.17 (m, 3H), 3.82 (s, 3H).

### Examples 11, 12 and 13

### (S)-2-(2-Amino-1-(3-chlorophenyl)ethyl)-6-(5-methyl-2-((1-methyl-1H-pyrazol-5-yl)a mino)pyrimidin-4-yl)-1H-pyrrolo[1,2-c]imidazol-3(2H)-one 11

### (R)-2-(1-(3-Chlorophenyl)ethyl)-6-(5-methyl-2-((1-methyl-1H-pyrazol-5-yl)amino)pyri midin-4-yl)-1H-pyrrolo[1,2-c]imidazol-3(2H-one 12

### (S)-2-(1-(3-Chlorophenyl)-2-(methylamino)ethyl)-6-(5-methyl-2-((1-methyl-1H-pyrazol -5-yl)amino)pyrimidin-4-yl)-1H-pyrrolo[1,2-c]imidazol-3(2H)-one 13

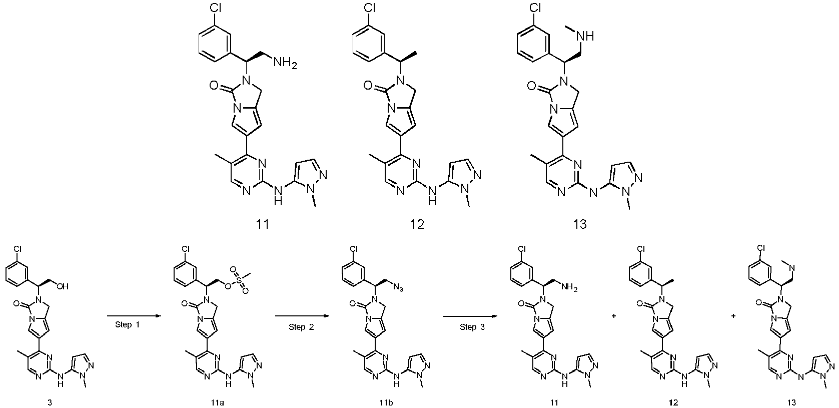

### Step 1

### (S)-2-(3-Chlorophenyl)-2-(6-(5-methyl-2-((1-methyl-1H-pyrazol-5-yl)amino)pyrimidin-4-yl)-3-oxo-1H-pyrrolo[1,2-c]imidazol-2(3H)-yl)ethyl methanesulfonate 11a

Compound **3** (12.0 mg, 0.026 mmol) was dissolved in 10 mL of dichloromethane, followed by the addition of triethylamine (8.0 mg, 0.079 mmol) and methanesulfonyl chloride (6.0 mg, 0.052 mmol) successively, and the reaction solution was stirred for 30 minutes. A small amount of water was added, and the reaction solution was concentrated under reduced pressure to obtain the title compound **11a** (14 mg), yield: 99%.

MS m/z (ESI): 542.0[M+1],

### Step 2

### (S)-2-(2-Azido-1-(3-chlorophenyl)ethyl)-6-(5-methyl-2-((1-methyl-1H-pyrazol-5-y 1)amino)pyrimidin-4-yl)-1H-pyrrolo[1,2-c]imidazol-3(2H)-one 11b

Compound **11a** (14 mg, 0.026 mmol) was dissolved in 7 mL of *N,N*-dimethylformamide, followed by the addition of sodium azide (8.4 mg, 0.130 mmol). After the addition was completed, the reaction solution was stirred for 3 hours at 70°C. Water was added, and the reaction solution was extracted with dichloromethane (10 mL×2). The organic phases were combined, wash withed saturated sodium chloride solution, dried over anhydrous sodium sulfate, filtered, and concentrated under reduced pressure to obtain compound **11b** (12 mg), yield: 95%.

MS m/z (ESI): 489.0[M+1].

### Step 3

### (S)-2-(2-Amino-1-(3-chlorophenyl)ethyl)-6-(5-methyl-2-((1-methyl-1H-pyrazol-5-yl)amino)pyrimidin-4-yl)-1H-pyrrolo[1,2-c]imidazol-3(2H)-one 11

### (R)-2-(1-(3-Chlorophenyl)ethyl)-6-(5-methyl-2-((1-methyl-1H-pyrazol-5-yl)amino)pyri midin-4-yl)-1H-pyrrolo[1,2-c]imidazol-3(2H)-one 12

### (S)-2-(1-(3-Chlorophenyl)-2-(methylamino)ethyl)-6-(5-methyl-2-((1-methyl-1H-pyrazol -5-yl)amino)pyrimidin-4-yl)-1H-pyrrolo[1,2-c]imidazol-3(2H)-one 13

Compound **11b** (17 mg, 0.035 mmol) was dissolved in 5 mL of methanol, followed by the addition of palladium on carbon (42 mg, 0.35 mmol, 10% palladium). The reaction system was purged with hydrogen three times, and stirred for 30 minutes. The reaction solution was filtered, concentrated under reduced pressure, and the residues were purified by preparative HPLC to obtain compound **11** (1.0 mg), yield: 6%, compound **12** (1.0mg), yield: 6%, and compound **13** (1.0 mg), yield: 6%.

### Compound 11

MS m/z (ESI): 463.2[M+1].

¹H NMR (400 MHz, CD₃OD): δ 8.23 (s, 1H), 7.75 (s, 1H), 7.60 (s, 1H), 7.52 (s, 1H), 7.47-7.42 (m, 3H), 6.74(s, 1H), 6.33 (s, 1H), 5.37-5.34 (dd, 1H), 4.56-4.52 (d, 1H), 4.36 (d, 1H), 4.26-4.16 (m, 1H), 4.14-4.02 (m, 1H), 3.76 (s, 3H), 2.39 (s, 3H).

### Compound 12

MS m/z (ESI): 448.1[M+1].

¹H NMR (400 MHz, CD₃OD): δ 8.23 (s, 1H), 7.74 (s, 1H), 7.60 (s, 1H), 7.53 (s, 1H), 7.45-7.34 (m, 3H), 6.75 (s, 1H), 6.42 (s, 1H), 5.45-5.40 (dd, 1H), 4.58-4.54 (d, 1H), 4.20-4.16 (d, 1H), 3.77 (s, 3H), 2.41 (s, 3H), 1.74-1.72 (d, 3H).

### Compound 13

MS m/z (ESI): 477.2[M+1].

¹H NMR (400MHz, CDCl₃) δ 8.15 (s, 1H), 7.93 (s, 1H), 7.58 (s, 1H), 7.46 (s, 1H), 7.42-7.27 (m, 4H), 6.61 (s, 1H), 6.28 (s, 1H), 5.39 (d, 1H), 4.41 (d, 1H), 4.21 (d, 1H), 3.81 (s, 3H), 3.68-3.52 (m, 2H), 2.35 (s, 3H), 2.07-2.01 (m, 3H).

### Example 14

### (S)-2-(1-(4-Chloro-3-fluorophenyl)-2-hydroxyethyl)-6-(5-methyl-2-((1-methyl-1H-pyra zol-5-yl)amino)pyrimidin-4-yl)-1H-pyrrolo[1,2-c]imidazol-3(2H)-one 14

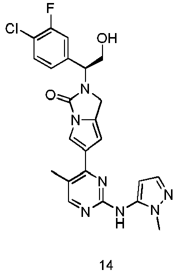

The synthetic route in Example 6 was applied with the replacement of the starting compound 6a in Step 1 with (*S*)-2-amino-2-(3-fluoro-4-chlorophenyl)ethan-1-ol, to obtain compound **14** (15 mg).

MS m/z (ESI): 482.2 [M+1].

¹H NMR (400 MHz, CDCl₃) δ 8.19 (s, 1H), 7.61-7.48 (m, 2H), 7.47-7.36 (m, 2H), 7.22 (d, 1H), 7.13 (d, 1H), 6.56 (s, 1H), 6.15 (s, 1H), 5.15 (dd, 1H), 4.42 (d, 1H), 4.32 (dd, 1H), 4.26 - 4.13 (m, 2H), 3.78 (s, 3H), 2.33 (s, 3H).

### Example 15

### (S)-2-(2-Hydroxy-1-(m-tolyl)ethyl)-6-(2-((1-methyl-1H-pyrazol-5-yl)amino)pyrimidin-4-yl)-1H-pyrrolo[1,2-c]imidazol-3(2H)-one 15

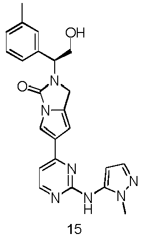

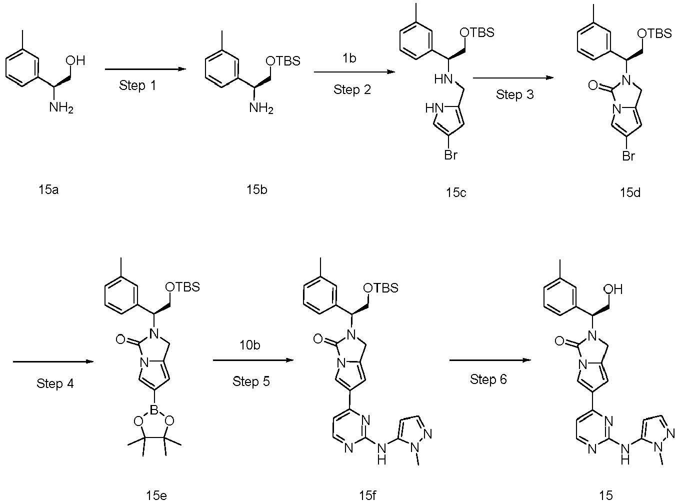

### Step 1

### (S)-2-((tert-Butyldimethylsilyl)oxy)-1-(m-tolyl)ethamine 15b

(*S*)-2-Amino-2-(*m*-tolyl)ethan-1-ol **15a** (3 g, 19.8 mmol, Shanghai Bide Pharmatech Ltd.) was dissolved in 100 mL of dichloromethane, followed by the addition of imidazole (4 g, 58.7 mol). The reaction solution was cooled to 0°C, followed by the addition of *tert*-butyldimethylchlorosilane (3.9 g, 25.9 mmol), and stirred for 14 hours. 100 mL of water was added, and the reaction solution was extracted with dichloromethane, dried over anhydrous sodium sulfate, and filtered. The filtrate was concentrated under reduced pressure, and the residues were purified by column chromatography with eluent system C to obtain the title compound **15b** (5 g), yield: 95%.

MS m/z (ESI): 266.2 [M+1].

### Step 2

### (S)-N-((4-Bromo-1H-pyrrol-2-yl)methyl)-2-((tert-butyldimethylsilyl)oxy)-1-(m-tolyl)et han-1-amine 15c

Compound **15b** (5 g, 19.5 mmol) and compound **1b** (3.4 g, 19.5 mmol) were stirred and reacted for 3 hours. 50 mL methanol was added, and the reaction solution was cooled to 0°C, followed by the addition of sodium borohydride (800 mg, 21.1 mmol) and stirred for 2 hours. Water was added, and the reaction solution was concentrated under reduced pressure. Water was added, and the reaction solution was extracted with ethyl acetate. The organic phases were combined, washed with saturated sodium chloride solution, dried over anhydrous sodium sulfate, and filtered. The filtrate was concentrated under reduced pressure, and the residues were purified by column chromatography with eluent system C to obtain the title compound **15c** (7.3 g), yield: 88%.

MS m/z (ESI): 423.1 [M+1].

### Step 3

### (S)-6-Bromo-2-(2-((tert-butyldimethylsilyl)oxy)-1-(m-tolyl)ethyl)-1H-pyrrolo[1,2-c]imi dazol-3(2H)-one 15d

Compound **15c** (7.3 g, 17.2 mmol) was dissolved in 150 mL of tetrahydrofuran, followed by the addition of *N,N*'-carbonyldiimidazole (8.4 g, 51.7 mmol) in an ice bath, and the reaction solution was stirred for 0.5 hours. Sodium hydride (60%, 2 g, 51.7 mmol) was added, and the reaction solution was stirred for 14 hours at room temperature, followed by the addition of saturated ammonium chloride solution. The reaction solution was concentrated under reduced pressure, and the residues were purified by column chromatography with eluent system C to obtain the title compound **15d** (7 g), yield: 90%.

MS m/z (ESI): 450.1 [M+1].

### Step 4

### (S)-2-(2-((tert-Butyldimethylsilyl)oxy)-1-(m-tolyl)ethyl)-6-(4,4,5,5-tetramethyl-1,3,2-di oxaborolan-2-yl)-1H-pyrrolo[1,2-c]imidazol-3(2H)-one 15e

Compound **15d** (7 g, 15.5 mmol) was dissolved in 100 mL of dioxane under argon atmosphere, followed by the addition of 4,4,4',4',5,5,5',5'-octamethyl-2,2'-bis(1,3,2-dioxaborolane) (5.9 g, 23.3 mmol), potassium acetate (3.1 g, 31.1 mmol) and [1,1'-bis(diphenylphosphino)ferrocene]palladium (II) dichloride (2.3 g, 2.1 mmol) successively. The reaction solution was stirred for 2 hours at 90°C, cooled, and filtered through Celite. The filtrate was concentrated, and the residues were purified by column chromatography with eluent system C to obtain the title compound **15e** (4 g), yield: 51.7%.

MS m/z (ESI): 497.2 [M+1].

### Step 5

### (S)-2-(2-((tert-Butyldimethylsilyl)oxy)-1-(m-tolyl)ethyl)-6-(2-((1-methyl-1H-pyraz ol-5-yl)amino)pyrimidin-4-yl)-1H-pyrrolo[1,2-c]imidazol-3(2H)-one 15f

A mixture of compound **10b** (550 mg, 2.62 mmol), compound **15e** (1.56 g, 3.15 mmol), [1,1'-bis(diphenylphosphino)ferrocene]palladium (II) dichloride (1.7 g, 0.26 mmol) and cesium carbonate (1.7 g, 5.2 mmol) was suspended in 30 mL of 1,4-dioxane and 6 mL of water under argon atmosphere. The reaction solution was heated to 80°C, and stirred for 14 hours. The reaction solution was cooled, and filtered through Celite. The filtrate was collected, and extracted with ethyl acetate (15 mL×2). The organic phases were combined and concentrated under reduced pressure, and the residues were purified by column chromatography with eluent system A to obtain the title compound **15f** (1.4 g), yield: 98%.

MS m/z (ESI): 544.2 [M+1].

### Step 6

### (S)-2-(2-Hydroxy-1-(m-tolyl)ethyl)-6-(2-((1-metbyl-1H-pyrazol-5-yl)amino)pyrimi din-4-yl)-1H-pyrrolo[1,2-c]imidazol-3(2H)-one 15

Compound **15f** (1.3 g, 2.4 mmol) was dissolved in 10 mL of dichloromethane, followed by the dropwise addition of 3 mL of trifluoroacetic acid. After the addition was completed, the reaction solution was stirred for 4 hours. The pH was adjusted to 7 with saturated sodium bicarbonate solution, and the reaction solution was extracted with dichloromethane (30 mL×2). The organic phases were combined and concentrated under reduced pressure, and the residues were purified by thin layer chromatography with developing solvent system A to obtain the compound **15** (500 mg), yield: 48.6%.

MS m/z (ESI): 430.1 [M+1].

¹H NMR (400 MHz, CD₃OD) δ 8.29 (d, 1H), 7.80 (s, 1H), 7.43 (d, 1H), 7.32-7.24 (m, 1H), 7.21 (s, 1H), 7.19-7.12 (m, 2H), 7.10 (d, 1H), 6.63 (s, 1H), 6.31 (d, 1H), 5.23 (dd, 1H), 4.61 (d, 1H), 4.29 (d, 1H), 4.19 (dd, 1H), 4.11-3.99 (m, 1H), 3.74 (s, 3H), 2.34 (s, 3H).

### Example 16

### (S)-2-(1-(3-Chloro-4-fluorophenyl)-2-hydroxyethyl)-6-(2-((1-methyl-1H-pyrazol-5 -yl)amino)pyrimidin-4-yl)-1H-pyrrolo[1,2-c]imidazol-3(2H)-one 16

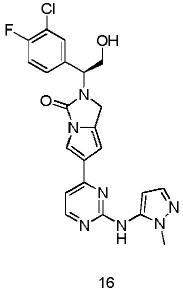

The synthetic route in Example 6 was applied with the replacement of the starting compound **If** in Step 5 with compound **10b**, to obtain compound **16** (20 mg).

MS m/z (ESI): 468.0 [M+1].

¹H NMR (400 MHz, CDCl₃) δ 8.31 (d, 1H), 7.69 (s, 1H), 7.51-7.42 (m, 2H), 7.33 (s, 1H), 7.26-7.22 (m, 1H), 7.21-7.13 (m, 1H), 6.90 (d, 1H), 6.49 (s, 1H), 6.30 (s, 1H), 5.14 (dd, 1H), 4.45 (d, 1H), 4.35-4.24 (m, 1H), 4.24-4.13 (m, 2H), 3.80 (s, 3H).

### Example 17

### (S)-2-(1-(3-Chlorophenyl)-2-hydroxyethyl)-6-(2-(isopropylamino)-5-methylpyrimidin-4 -yl)-1H-pyrrolo[1,2-c]imidazol-3(2H)-one 17

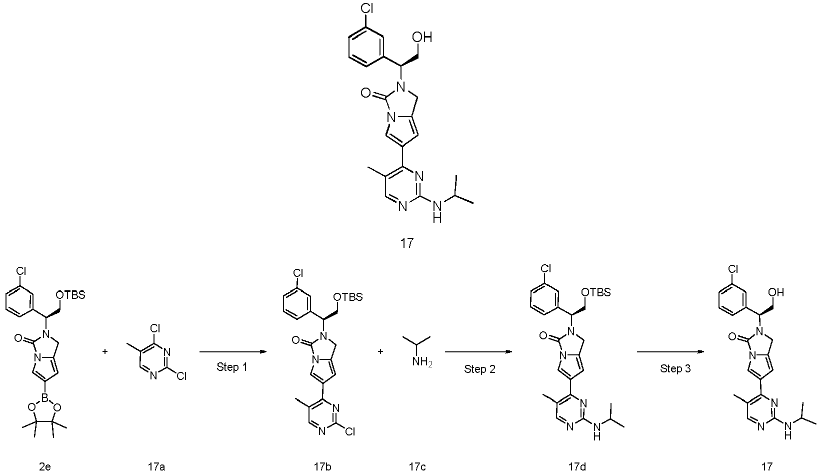

### Step 1

### (S)-2-(2-((tert-Butyldimethylsilyl)oxy)-1-(3-chlorophenyl)ethyl)-6-(2-chloro-5-methylp yrimidin-4-yl)-1H-pyrrolo[1,2-c]imidazol-3(2H)-one 17b

A mixture of 2,4-dichloro-5-methylpyrimidine **17a** (76.3 mg, 0.47 mmol, Shanghai Bide Pharmatech Ltd.), compound **2e** (220 mg, 0.43 mmol), [1,1'-bis(diphenylphosphino)ferrocene]palladium (II) dichloride (62.3 mg, 0.08 mmol) and cesium carbonate (277.3 mg, 0.85 mmol) was suspended in 10 mL of 1,4-dioxane and 2 mL of water under argon atmosphere. The reaction solution was heated to 80°C, and stirred for 14 hours. The reaction solution was cooled, and filtered through Celite. The filtrate was collected, and extracted with ethyl acetate (10 mL×3). The organic phases were combined and concentrated, and the residues were purified by column chromatography with eluent system A to obtain the title compound **17b** (100 mg), yield: 45.4%.

MS m/z (ESI): 517.1 [M+1].

### Step 2

### (S)-2-(2-((tert-Butyldimethylsilyl)oxy)-1-(3-chlorophenyl)ethyl)-6-(2-(isopropylamino) -5-methylpyrimidin-4-yl)-1H-pyrrolo[1,2-c]imidazol-3(2H)-one 17d

Compound **17b** (80 mg, 154.5 µmol) and isopropylamine **17c** (91.4 mg, 1.5 mmol, Shanghai Bide Pharmatech Ltd.) were dissolved in 2 mL of *N,N*-dimethylacetamide, and the reaction solution was stirred for 6 hours at 150°C in microwave reactor. The reaction solution was concentrated under reduced pressure, and the residues were purified by column chromatography with eluent system A to obtain the title compound **17d** (20 mg), yield: 24%.

### Step 3

### (S)-2-(1-(3-Chlorophenyl)-2-hydroxyethyl)-6-(2-(isopropylamino)-5-methylpyrimidin-4 -yl)-1H-pyrrolo[1,2-c]imidazol-3(2H)-one 17

Compound **17d** (20 mg, 37 µmol) was dissolved in 5 mL of dichloromethane, followed by the addition of 0.5 mL of trifluoroacetic acid dropwise. After the addition was completed, the reaction solution was stirred for 1 hour. The pH was adjusted to 7 with saturated sodium bicarbonate solution, and the reaction solution was extracted with dichloromethane (20 mL×2). The organic phases were combined and concentrated, and the residues were purified by column chromatography with eluent system A to obtain the title compound **17** (5 mg), yield: 32%.

MS m/z (ESI): 426.2 [M+1].

¹H NMR (400 MHz, CDCl₃) δ 8.08 (s, 1H), 7.61 (s, 1H), 7.34 (d, 3H), 7.25 (d, 2H), 6.70 (s, 1H), 5.13 (dd, 1H), 4.45 (d, 1H), 4.36-4.25 (m, 2H), 4.23-4.14 (m, 2H), 2.30 (s, 3H), 1.26 (d, 6H).

### Example 18

### 2-(3,4-Difluorobenzyl)-6-(5-methyl-2-((1-methyl-1H-pyrazol-5-yl)amino)pyrimidin-4-y 1)-1H-pyrrolo[1,2-c]imidazol-3(2H)-one 18

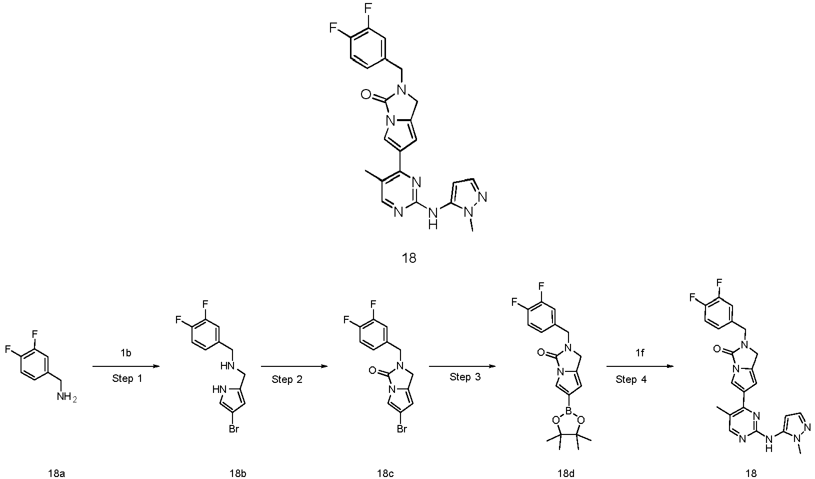

### Step 1

### 1-(4-Bromo-1H-pyrrol-2-yl)-N-(3,4-difluorobenzyl)formamide 18b

Compound (3,4-difluorophenyl)methanamine **18a** (863 mg, 6.0 mmol) and compound **1b** (1 g, 5.7 mmol) were stirred and reacted for 3 hours. 20 mL of methanol was added, and the reaction solution was cool to 0°C. Sodium borohydride (361 mg, 5.7 mmol) was added, and the reaction solution was stirred for 2 hours. Water was added, and the reaction solution was concentrated under reduced pressure. Water was added, and the reaction solution was extracted with ethyl acetate. The organic phases were combined, washed with saturated sodium chloride solution, dried over anhydrous sodium sulfate, and filtered. The filtrate was concentrated under reduced pressure, and the residues were purified by column chromatography with eluent system C to obtain the title compound **18b** (1.7 g), yield: 98.2%.

MS m/z (ESI): 301.0 [M+1].

### Step 2

### 6-Bromo-2-(3,4-difluorobenzyl)-1H-pyrrolo[1,2-c]imidazol-3(2H)-one 18c

Compound **18b** (0.5 g, 1.7 mmol) was dissolved in 50 mL of tetrahydrofuran, followed by the addition of *N,N*'-carbonyldiimidazole (286 mg, 2.0 mmol) in an ice bath, and the reaction solution was stirred for 0.5 hours. Sodium hydride (60%, 15 mg, 0.63 mmol) was added, and the reaction solution was stirred for 14 hours at room temperature, followed by the addition of saturated ammonium chloride solution. The reaction solution was concentrated under reduced pressure, and the residues were purified by column chromatography with eluent system C to obtain the title compound **18c** (220 mg), yield: 40.5%.

MS m/z (ESI): 327.0 [M+1].

### Step 3

### 2-(3,4-Difluorobenzyl)-6-(4,4,5,5-tetramethyl-1,3,2-dioxaborolan-2-yl)-1H-pyrrolo[1,2-c]imidazol-3(2H)-one 18d

Compound **18c** (100 mg, 0.31 mmol) was dissolved in 10 mL of dioxane under argon atmosphere, followed by the addition of 4,4,4',4',5,5,5',5'-octamethyl-2,2'-bis(1,3,2-dioxaborolane) (95 mg, 0.37 mmol), potassium acetate (60 mg, 0.61 mmol) and [1,1'-bis(diphenylphosphino)ferrocene]palladium (II) dichloride (22 mg, 30 µmol) successively. The reaction solution was stirred for 2 hours at 90°C, cooled, and filtered through Celite. The filtrate was concentrated, and the residues were purified by column chromatography with eluent system C to obtain the title compound **18d** (28 mg), yield: 24.5%.

MS m/z (ESI): 375.0 [M+1].

### Step 4

### 2-(3,4-Difluorobenzyl)-6-(5-methyl-2-((1-methyl-1H-pyrazol-5-yl)amino)pyrimidin-4-y 1)-1H-pyrrolo[1,2-c]imidazol-3(2H)-one 18

A mixture of compound **18d** (27 mg, 72 µmol), compound **If** (15 mg, 67 µmol), [1,1'-bis(diphenylphosphino)ferrocene]palladium (II) dichloride (6 mg, 8.2 µmol) and cesium carbonate (18 mg, 130 µmol) was suspended in 5 mL of 1,4-dioxane and 1 mL of water under argon atmosphere. The reaction solution was heated to 80°C, and stirred for 14 hours. The reaction solution was cooled, and filtered through Celite. The filtrate was collected, and extracted with ethyl acetate (10 mL×2). The organic phases were combined and concentrated under reduced pressure, and the residues were purified by column chromatography with eluent system A to obtain the title compound 18 (10 mg), yield: 34%.

MS m/z (ESI): 435.9 [M+1].

¹H NMR (400 MHz, CD₃OD) δ 8.23 (s, 1H), 7.80 (s, 1H), 7.63-7.54 (m, 1H), 7.36-7.22 (m, 2H), 7.19 (s, 1H), 6.75 (s, 1H), 6.48 (d, 1H), 4.69 (s, 2H), 4.41 (s, 2H), 3.80 (s, 3H), 2.42 (s, 3H).

### Example 19

### (S)-2-(1-(3-Fluorophenyl)-2-hydroxyethyl)-6-(2-((1-methyl-1H-pyrazol-5-yl)amino)pyri midin-4-yl)-1H-pyrrolo[1,2-c]imidazol-3(2H)-one 19

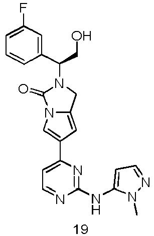

The synthetic route in Example 15 was applied with the replacement of the starting compound (*S*)-2-amino-2-(3-methylphenyl)ethan-1-ol **15a** in Step 1 with (*S*)-2-amino-2-(3-fluorophenyl)ethan-1-ol, to obtain compound **19** (50 mg).

MS m/z (ESI): 434.1 [M+1].

¹H NMR (400 MHz, CD₃OD) δ 8.29 (d, 1H), 7.81 (s, 1H), 7.51-7.34 (m, 2H), 7.28-7.15 (m, 2H), 7.14-6.99 (m, 2H), 6.65 (s, 1H), 6.32 (d, 1H), 5.26 (dd, 1H), 4.64 (d, 1H), 4.34 (d, 1H), 4.26-4.14 (m, 1H), 4.13-4.03 (m, 1H), 3.75 (s, 3H).

### Example 20

### (S)-2-(1-(3-Fluoro-4-chlorophenyl)-2-hydroxyethyl)-6-(2-((1-methyl-1H-pyrazol-5-yl)a mino)pyrimidin-4-yl)-1H-pyrrolo[1,2-c]imidazol-3(2H)-one 20

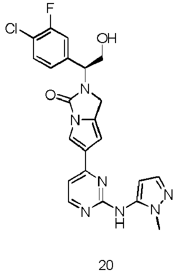

The synthetic route in Example 14 was applied with the replacement of the starting compound **If** in Step 5 with compound **10b**, to obtain compound **20** (50 mg).

MS m/z (ESI): 468.1 [M+1].

¹H NMR (400MHz, CD₃OD) δ 8.29 (d, 1H), 7.81 (s, 1H), 7.49 (t, 1H), 7.44 (d, 1H), 7.35 (dd, 1H), 7.22 (d, 1H), 7.10 (d, 1H), 6.65 (s, 1H), 6.32 (d, 1H), 5.24 (dd, 1H), 4.64 (d, 1H), 4.36 (d, 1H), 4.21-4.13 (m, 1H), 4.11-4.03 (m, 1H), 3.75 (s, 3H).

### Example 21

### (S)-2-(1-(4-Chlorophenyl)-2-hydroxyethyl)-6-(2-((1-methyl-1H-pyrazol-5-yl)amino)pyr imidin-4-yl)-1H-pyrrolo[1,2-c]imidazol-3(2H)-one 21

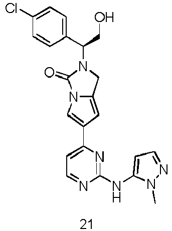

The synthetic route in Example 15 was applied with the replacement of the starting compound **15a** in Step 1 with (*S*)-2-amino-2-(4-chlorophenyl)ethan-1-ol, to obtain compound **21** (10 mg).

MS m/z (ESI): 450.1 [M+1].

¹H NMR (400 MHz, CDCl₃) δ 8.33 (d, 1H), 7.75 (s, 1H), 7.48 (d, 1H), 7.42-7.35 (m, 2H), 7.31 (d, 2H), 6.94 (d, 1H), 6.54 (s, 1H), 6.35 (s, 1H), 5.14 (dd, 1H), 4.44 (d, 1H), 4.30 (dd, 1H), 4.25-4.14 (m, 2H), 3.82 (s, 3H).

### Biological Assay

Test example 1: ERK1 enzyme activity test

### 1. Test purpose

The purpose of this experiment is to detect the inhibitory ability of the compounds on the ERK1 enzyme activity, and evaluate the *in vitro* activity of the compounds based on the IC₅₀. The ADP-Glo^{™} Kinase Assay Kit is used in this experiment. Under the action of the enzyme, the substrate is phosphorylated and ADP is produced at the same time. The ADP-Glo reagent is added to remove the unreacted ATP in the reaction system, and the ADP produced by the reaction is detected with the kinase detection reagent. In the presence of the compound, the inhibition rate of the compound is calculated by measuring the signal value.

### 2. Experimental method

Formulation of enzyme and substrate: ERK1 (1879-KS-010, R&D) and substrate (AS-61777, anaspec) were formulated into 0.75 ng/µl and 100 µL respectively in the buffer (40 mM Tris, 20 mM MgCl2, 0.1 mg/ml BSA, 50 µM DTT), and then the enzyme solution and the substrate solution were prepared into a mixed solution at a volume ratio of 2:1 for later use. ATP was diluted to 300 µM with the buffer. The compound was dissolved in DMSO to prepare a stock solution with an initial concentration of 20 mM, and then Bravo (SGC120TH34702, Agilent Technologies) was used to dilute the stock solution to desired concentrations. Finally, a 3µL of a mixed solution of enzyme and substrate, and 1µL of different concentrations of the compound (the initial concentration is 50 µM, 4-fold dilution) were added to each well of the 384-well plate, and the plate was incubated at 30°C for 10 minutes, and finally 1µL of 300 µM ATP solution was added to each well, and the plate was incubated at 30°C for 2 hours. Then 5 µL of ADP-Glo was added, and the plate was incubated at 30°C for 40 minutes. Then 10 µL of Kinase detection buffer was added, and the plate was incubated at 30°C for 40 minutes. The 384-well plate was taken out and placed in a microplate reader (BMG labtech, PHERAstar FS), and the chemiluminescence was measured by the microplate reader.

### 3. Data analysis

Microsoft Excel, Graphpad Prism 5 was used to process and analyze the data. The IC₅₀ value of the compound was obtained, and the results are shown in Table 1 below.

**Table 1 The IC₅₀ value of the inhibition of the present compounds on ERK1 enzyme activity**

| Example No. | IC₅₀ (nM) |
|---|---|
| 1 | 9 |
| 3 | 3 |
| 5 | 10 |
| 6 | 14 |
| 9 | 12 |
| 10 | 5 |
| 13 | 2 |
| 15 | 5 |
| 16 | 70 |
| 19 | 24 |
| 20 | 30 |
| 21 | 67 |

Conclusion: The compounds of the present disclosure have a significant inhibitory effect on the ERK1 enzyme activity.

### Test example 2: ERK2 enzyme activity test

### 1. Test purpose

The purpose of this experiment is to detect the inhibitory ability of the compounds on the ERK2 enzyme activity, and evaluate the in vitro activity of the compounds based on the IC₅₀. The ADP-Glo^{™} Kinase Assay Kit is used in this experiment. Under the action of the enzyme, the substrate is phosphorylated and ADP is produced at the same time. The ADP-Glo reagent is added to remove the unreacted ATP in the reaction system, and the ADP produced by the reaction is detected by the kinase detection reagent. In the presence of the compound, the inhibition rate of the compound is calculated by measuring the signal value.

### 2. Experimental method

Formulation of enzyme and substrate: ERK2 (1879-KS-010, R&D) and substrate (custom peptide, Gill Biochemical) were formulated into 0.75 ng/µl and 1500 ng in the buffer (40 mM Tris, 20 mM MgCl2, 0.1 mg/ml BSA, 50 µM DTT), and then the enzyme solution and the substrate solution were prepared into a mixed solution at a volume ratio of 2:1 for later use. ATP was diluted to 500 µM with the buffer. The compound was dissolved in DMSO to prepare a stock solution with an initial concentration of 20 mM, and then Bravo (SGC120TH34702, Agilent Technologies) was used to dilute the stock solution to desired concentrations. Finally, a 3µL of a mixed solution of enzyme and substrate, and 1µL of different concentrations of the compound (the initial concentration is 50 µM, 4-fold dilution) were added to each well of the 384-well plate, and the plate was incubated at 30°C for 10 minutes, and finally 1µL of 500 µM ATP solution was added to each well, and the plate was incubated at 30°C for 2 hours. Then 5 µL of ADP-Glo was added, and the plate was incubated at 30°C for 40 minutes. Then 10 µL of kinase detection buffer was added, and the plate was incubated at 30°C for 40 minutes. The 384-well plate was taken out and placed in a microplate reader (BMG labtech, PHERAstar FS), and the chemiluminescence was measured by the microplate reader.

### 3. Data analysis

Microsoft Excel, Graphpad Prism 5 was used to process and analyze the data. The IC₅₀ value of the compound was obtained, and the results are shown in Table 2 below.

**Table 2 The IC₅₀ value of the inhibition of the present compounds on ERK2 enzyme activity**

| Example No. | IC₅₀ (nM) |
|---|---|
| 1 | 2 |
| 2 | 3 |
| 3 | 2 |
| 4 | 6 |
| 5 | 7 |
| 6 | 10 |
| 7 | 63 |
| 8 | 39 |
| 9 | 6 |
| 10 | 7 |
| 11 | 18 |
| 12 | 45 |
| 13 | 2 |
| 14 | 5 |
| 15 | 5 |
| 16 | 44 |
| 18 | 16 |
| 19 | 20 |
| 20 | 18 |
| 21 | 45 |

Conclusion: The compounds of the present disclosure have a significant inhibitory effect on the ERK2 enzyme activity.

### Test example 3: In Vitro Proliferation Inhibition Test of Compounds on Colo205 Tumor Cells

### 1. Test purpose

The purpose of this experiment is to test the inhibitory activity of the compounds on the proliferation of Colo205 cells (CCL-222, ATCC) *in vitro.* The cells were treated *in vitro* with different concentrations of the compounds. After 3 days of culture, the cell proliferation was tested with CTG (CellTiter-Glo^{®} Luminescent Cell Viability Assay, Promega, Catalog No. G7573) reagent, and the *in vitro* activity of the compounds was evaluated according to the IC₅₀ value.

### 2. Experimental method

In the following, taking the *in vitro* proliferation inhibition test method of Colo205 cells as an example, the method in the present disclosure for testing the *in vitro* proliferation inhibitory activity of the compounds of the present disclosure is described. This method is also applicable to, but not limited to, the *in vitro* proliferation inhibitory activity test on other tumor cells.

Colo205 cells were digested, centrifuged and then resuspended. The single cell suspension was mixed well, and the density of viable cells was adjusted to 5.0×10⁴ cells/ml with cell culture medium (RPMI1640+2% FBS), and 95µl/well was added to a 96-well cell culture plate. Only 100 µl medium was added to the peripheral wells of the 96-well plate. The culture plate was incubated in an incubator for 24 hours (37°C, 5% CO₂)·

The compound was dissolved in DMSO and prepared into a stock solution with an initial concentration of 20 mM. The initial concentration of the small molecule compound was 2 mM, and then 4-fold diluted into 9 points, and the 10^{th} point is DMSO. Another 96-well plate was taken and 90 µl of cell culture medium (RPMI1640+2% FBS) was added to each well, then 10 µl of different concentrations of the test sample was added to each well. The mixture was mixed well, and then 5uL of different concentrations of the test sample was added to the cell culture plate with duplicate well for each sample. The culture plate was incubated in an incubator for 3 days (37°C, 5% CO₂). The 96-well cell culture plate was taken out, 50µL CTG solution was added to each well, and the plate was incubated for 10 minutes at room temperature. In a microplate reader (BMG labtech, PHERAstar FS), chemiluminescence was measured with the microplate reader.

### 3. Data analysis

Microsoft Excel, Graphpad Prism 5 was used to process and analyze the data. The example results are shown in Table 3 below.

**Table 3 The IC₅₀ value of the inhibition of the present compounds on in vitro Colo205 tumor cell proliferation**

| Example No. | IC₅₀ (nM) |
|---|---|
| 1 | 90 |
| 2 | 32 |
| 3 | 26 |
| 4 | 82 |
| 5 | 81 |
| 6 | 74 |
| 7 | 158 |
| 9 | 77 |
| 10 | 62 |
| 13 | 99 |
| 14 | 92 |
| 15 | 55 |
| 16 | 180 |
| 19 | 101 |
| 20 | 68 |
| 21 | 120 |

### Pharmacokinetics Evaluation

### Test Example 4. Pharmacokinetics assay of the compounds of the present disclosure in mice

### 1. Abstract

Mice were used as test animals. The drug concentration in plasma at different time points was determined by LC/MS/MS method after intragastrical administration of the compounds of Example 3, Example 10, Example 15 and Example 20 to mice. The pharmacokinetic behavior of the compounds of the present disclosure was studied in mice , and the pharmacokinetic characteristics were evaluated.

### 2. Test protocol

### 2.1 Test compounds

Compounds of Example 3, Example 10, Example 15 and Example 20.

### 2.2 Test animals

Thirty-six C57 mice (female, equally divided into 4 groups) were purchased from Shanghai Jiesijie Laboratory Animal Co., LTD. (Certificate No.: SCXK(Shanghai)2013-0006).

### 2.3 Preparation of the test compound

A certain amount of the test compound was weighed and dissolved by the addition of 5% of DMSO and 5% of tween 80. Then 90% of normal saline were added to prepare a 0.1 mg/mL colorless, clear and transparent solution.

### 2.4 Administration

After an overnight fast, C57 mice were intragastrically administered the test compound at a dosage of 2 mg/kg and a volume of 0.2ml/10g.

### 3. Process

The mice were intragastrically administered the test compounds. 0.1 ml of blood was taken before the administration and at 0.25, 0.5, 1.0, 2.0, 4.0, 6.0, 8.0, 11.0 and 24.0 hours after the administration. The samples were stored in heparinized tubes, and centrifuged for 10 minutes at 3500 rpm to separate the blood plasma. The plasma samples were stored at -20°C.

The content of the test compound in the plasma of mice after intragastrical administration of the test compound at different concentrations was determined: 25 µL of rat plasma at each time point after the administration was taken, followed by the addition of 50 µL of the internal standard camptothecin solution (National Institutes for Food and Drug Control of China) and 200 µL of acetonitrile. The resulting solution was vortex-mixed for 5 minutes, and centrifuged for 10 minutes (4000 rpm). 4 µL of the supernatant was taken from the plasma samples for LC/MS/MS analysis.

### 4. Results of pharmacokinetic parameters

Pharmacokinetic parameters of the compounds of the present disclosure are shown below:

| Example No. | Pharmacokinetics assay in mice | | | | | |
|---|---|---|---|---|---|---|
| | Plasma concentration | Area under curve | Half-life | Residence time | Clearance | Apparent distribution volume |
| | Cmax (ng /mL) | AUC (ng /mL^{∗}h) | T1/2 (h) | MRT (h) | CLz/F (ml/min/kg) | Vz/F (ml/kg) |
| Example 3 | 604 | 2472 | 3.51 | 4.05 | 13.5 | 4097 |
| Example 10 | 1023 | 3441 | 3.66 | 3.47 | 9.69 | 3067 |
| Example 15 | 816 | 3705 | 2.74 | 3.3 | 9 | 2134 |
| Example 20 | 1540 | 7632 | 3.73 | 4.95 | 4.37 | 1411 |

Conclusion: The compounds of the present disclosure are well absorbed, and have a significant pharmacokinetic advantage.

## Claims

1. A compound of general formula (I): 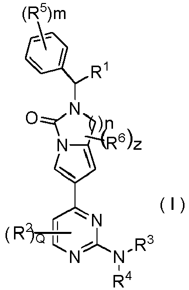
or a stereoisomer, tautomer, mesomer, racemate, enantiomer, diastereomer thereof, or mixture thereof, or a pharmaceutically acceptable salt thereof,
wherein:
R¹ is selected from the group consisting of hydrogen atom, halogen, alkyl, alkoxy, haloalkyl, haloalkoxy, hydroxy, hydroxyalkyl, cyano, amino, aminoalkyl and nitro, wherein the alkyl is optionally substituted by one or more substituents selected from the group consisting of NR⁷R⁸, alkoxy, halogen, cyano, nitro, hydroxy and hydroxyalkyl;
R² is identical or different and each is independently selected from the group consisting of hydrogen atom, halogen, alkyl, alkoxy, haloalkyl, haloalkoxy, hydroxy, hydroxyalkyl, cyano, amino and nitro;
R³ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁴ is selected from the group consisting of hydrogen atom, halogen, alkyl, alkoxy, haloalkyl, haloalkoxy, hydroxy, hydroxyalkyl, cyano, amino and nitro;
R⁵ is identical or different and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁶ is selected from the group consisting of hydrogen atom, halogen, alkyl, alkoxy, haloalkyl, haloalkoxy, hydroxy, hydroxyalkyl, cyano, amino and nitro;
R⁷ and R⁸ are identical or different and are each independently selected from the group consisting of hydrogen atom, alkyl, hydroxyalkyl and haloalkyl;
m is selected from the group consisting of 0, 1, 2, 3, 4 and 5;
n is selected from the group consisting of 0, 1, 2 and 3;
z is selected from the group consisting of 0, 1, 2, 3 and 4; and
Q is selected from the group consisting of 0, 1 and 2.

2. The compound of general formula (I) or the stereoisomer, tautomer, mesomer, racemate, enantiomer, diastereomer thereof, or mixture thereof, or the pharmaceutically acceptable salt thereof according to claim 1, wherein R¹ is selected from the group consisting of hydrogen atom, halogen, alkyl, alkoxy, haloalkyl, haloalkoxy, hydroxy, hydroxyalkyl, cyano, amino, aminoalkyl and nitro.

3. The compound of general formula (I) according to claims 1 or 2, being a compound of general formula (I-P): 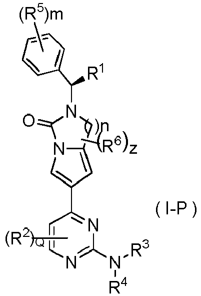
or a stereoisomer, tautomer, mesomer, racemate, enantiomer, diastereomer thereof, or mixture thereof, or a pharmaceutically acceptable salt thereof,
wherein R¹ to R⁶, m, n, z and Q are as defined in claims 1 or 2.

4. The compound of formula (I) or the stereoisomer, tautomer, mesomer, racemate, enantiomer, diastereomer thereof, or mixture thereof, or the pharmaceutically acceptable salt thereof according to any one of claims 1 to 3, wherein R⁴ is hydrogen atom.

5. The compound of formula (I) or the stereoisomer, tautomer, mesomer, racemate, enantiomer, diastereomer thereof, or mixture thereof, or the pharmaceutically acceptable salt thereof according to any one of claims 1 to 4, wherein n is 1 or 2.

6. The compound of formula (I) according to claims 1 or 2, being a compound of formula (II) or formula (II-P): 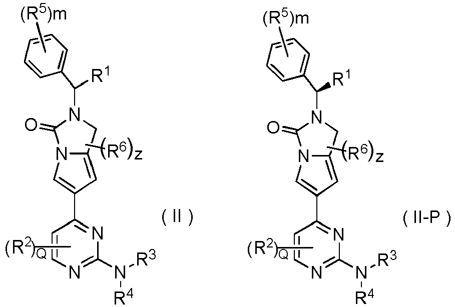
or a stereoisomer, tautomer, mesomer, racemate, enantiomer, diastereomer thereof, or mixture thereof, or a pharmaceutically acceptable salt thereof,
wherein R¹ to R⁶, m, z and Q are as defined in claims 1 or 2.

7. The compound of formula (I) or the stereoisomer, tautomer, mesomer, racemate, enantiomer, diastereomer thereof, or mixture thereof, or the pharmaceutically acceptable salt thereof according to any one of claims 1 to 6, wherein R¹ is selected from the group consisting of hydrogen atom, alkyl, hydroxy, aminoalkyl, alkylaminoalkyl and hydroxyalkyl; preferably, R¹ is selected from the group consisting of hydrogen atom, C₁-₆ alkyl, hydroxy, aminoC₁₋₆ alkyl, C₁-₆ alkylaminoC₁₋₆ alkyl and C₁-₆ hydroxyalkyl; and more preferably, R¹ is selected from the group consisting of hydrogen atom, methyl, hydroxymethyl, aminomethyl and methylaminomethyl.

8. The compound of formula (I) or the stereoisomer, tautomer, mesomer, racemate, enantiomer, diastereomer thereof, or mixture thereof, or the pharmaceutically acceptable salt thereof according to any one of claims 1 to 7, wherein R¹ is selected from the group consisting of hydrogen atom, alkyl, hydroxy, aminoalkyl and hydroxyalkyl; preferably, R¹ is selected from the group consisting of hydrogen atom, C₁₋₆ alkyl, hydroxy, aminoC₁₋₆ alkyl and C₁₋₆ hydroxyalkyl; and more preferably, R¹ is selected from the group consisting of hydrogen atom, methyl, hydroxymethyl and aminomethyl.

9. The compound of formula (I) according to claims 1 or 2, being a compound of formula (III) or formula (III-P): 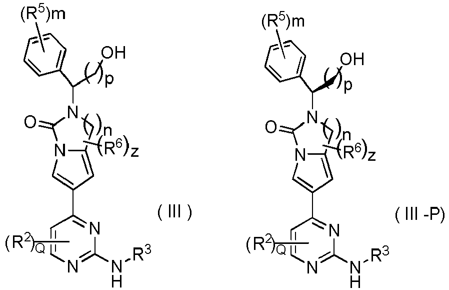
or a stereoisomer, tautomer, mesomer, racemate, enantiomer, diastereomer thereof, or mixture thereof, or a pharmaceutically acceptable salt thereof,
wherein:
p is selected from the group consisting of 0, 1, 2 and 3, and preferably 1;
R², R³, R⁵, R⁶, m, n, z and Q are as defined in claims 1 or 2.

10. The compound of formula (I) or the stereoisomer, tautomer, mesomer, racemate, enantiomer, diastereomer thereof, or mixture thereof, or the pharmaceutically acceptable salt thereof according to any one of claims 1 to 9, wherein R² is selected from the group consisting of hydrogen atom, halogen and alkyl; preferably, R² is selected from the group consisting of hydrogen atom, halogen and C₁₋₆ alkyl; and more preferably, R² is C₁₋₆ alkyl.

11. The compound of formula (I) or the stereoisomer, tautomer, mesomer, racemate, enantiomer, diastereomer thereof, or mixture thereof, or the pharmaceutically acceptable salt thereof according to any one of claims 1 to 10, wherein R³ is selected from the group consisting of alkyl, hydroxyalkyl, cycloalkyl, heterocyclyl and heteroaryl, wherein the alkyl, cycloalkyl, heterocyclyl and heteroaryl are each optionally further substituted by one or more substituents selected from the group consisting of alkyl, alkoxy, oxo, halogen, amino, cyano, nitro, hydroxy and hydroxyalkyl; and preferably, R³ is selected from the group consisting of C₁₋₆ alkyl, C₁₋₆ hydroxyalkyl, C₃₋₆ cycloalkyl, 3 to 8 membered heterocyclyl and 5 to 10 membered heteroaryl, wherein the C₁₋₆ alkyl, C₃₋₆ cycloalkyl, 3 to 8 membered heterocyclyl and 5 to 10 membered heteroaryl are each optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl.

12. The compound of formula (I) or the stereoisomer, tautomer, mesomer, racemate, enantiomer, diastereomer thereof, or mixture thereof, or the pharmaceutically acceptable salt thereof according to any one of claims 1 to 11, wherein R³ is a heteroaryl, wherein the heteroaryl is optionally further substituted by one or more substituents selected from the group consisting of alkyl, alkoxy, oxo, halogen, amino, cyano, nitro, hydroxy and hydroxyalkyl; preferably, R³ is a 5 to 10 membered heteroaryl, wherein the 5 to 10 membered heteroaryl is optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl; and preferably, R³ is a pyrazolyl, wherein the pyrazolyl is optionally substituted by C₁₋₆ alkyl, and preferably methyl.

13. The compound of formula (I) or the stereoisomer, tautomer, mesomer, racemate, enantiomer, diastereomer thereof, or mixture thereof, or the pharmaceutically acceptable salt thereof according to any one of claims 1 to 12, wherein R⁵ is selected from the group consisting of hydrogen atom, alkyl, alkoxy and halogen; and preferably, R⁵ is selected from the group consisting of hydrogen atom, C₁₋₆ alkyl, C₁₋₆ alkoxy and halogen.

14. The compound of formula (I) or the stereoisomer, tautomer, mesomer, racemate, enantiomer, diastereomer thereof, or mixture thereof, or the pharmaceutically acceptable salt thereof according to any one of claims 1 to 13, wherein R⁶ is a hydrogen atom.

15. The compound of formula (I) or the stereoisomer, tautomer, mesomer, racemate, enantiomer, diastereomer thereof, or mixture thereof, or the pharmaceutically acceptable salt thereof according to any one of claims 1 to 14, selected from the group consisting of: 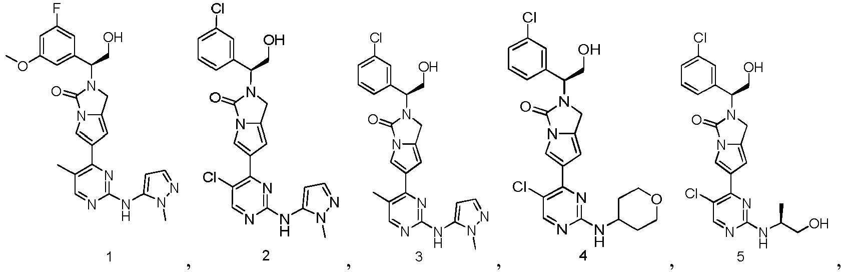 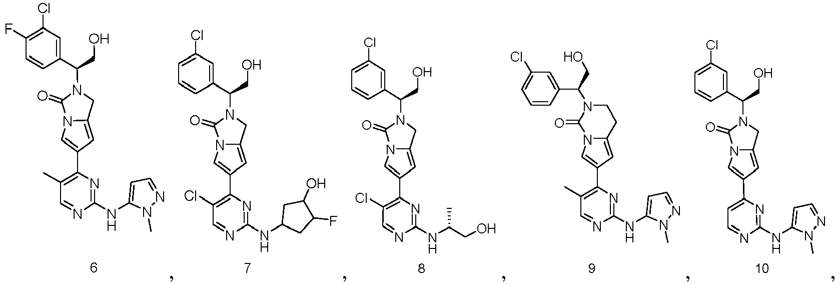 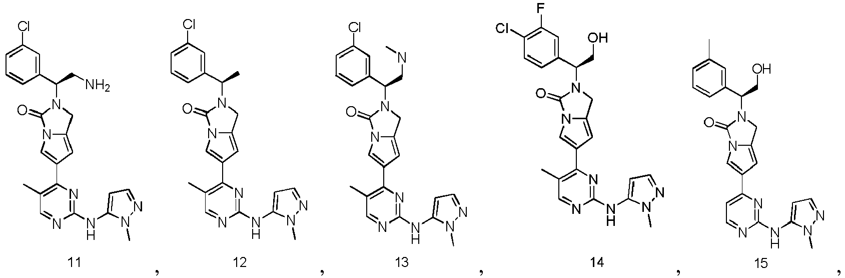 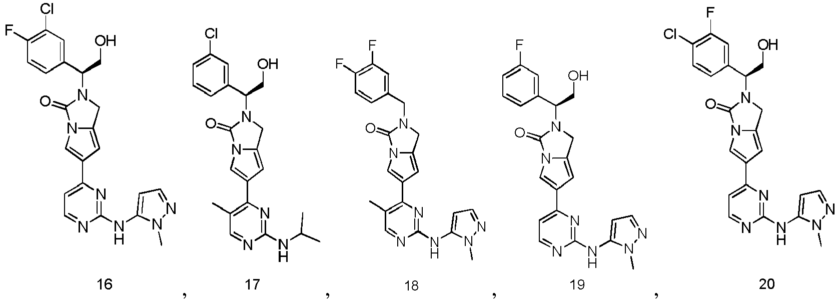 and

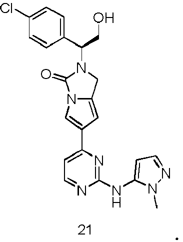

16. A compound of formula (IIIA) or formula (III-PA): 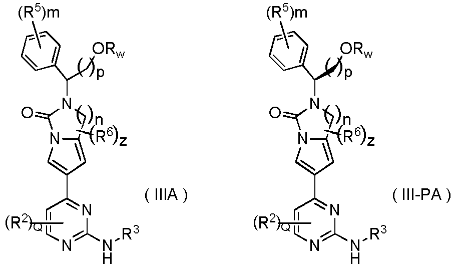
or a stereoisomer, tautomer, mesomer, racemate, enantiomer, diastereomer thereof, or mixture thereof, or a pharmaceutically acceptable salt thereof,
wherein:
R_{w} is a hydroxy protecting group; and
R², R³, R⁵, R⁶, m, n, p, Q and z are as defined in claim 9.

17. The compound of formula (IIIA) or formula (III-PA) according to claim 16, selected from the group consisting of:

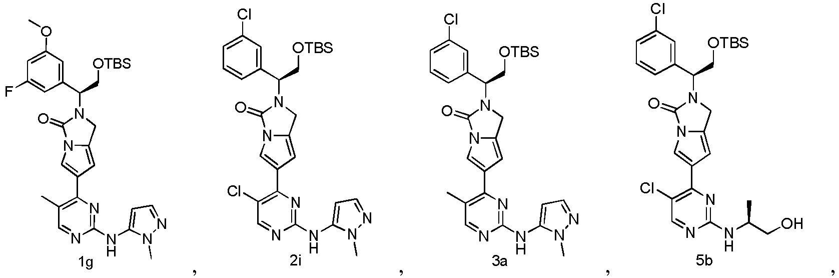

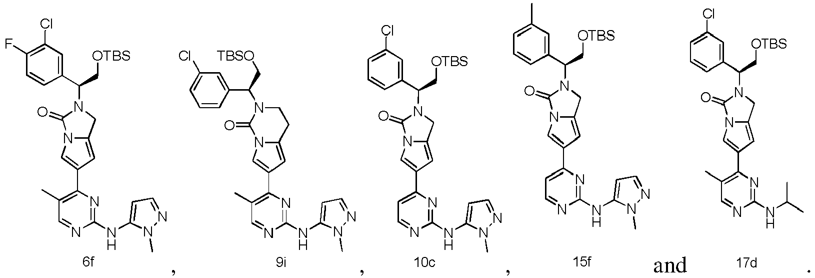

18. A method for preparing the compound of formula (III) or formula (III-P) according to claim 9, comprising a step of: 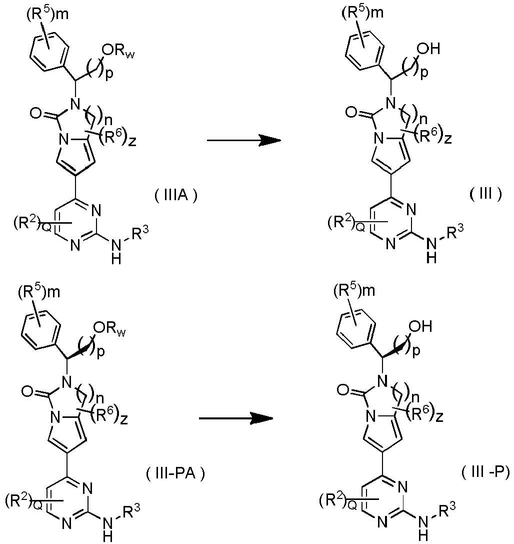
removing the hydroxy protecting group R_{w} from a compound of formula (IIIA) or formula (III-PA) under an acidic condition to obtain the compound of formula (III) or formula (III-P);
wherein:
the hydroxy protecting group R_{w} is preferably TBS; and
R², R³, R⁵, R⁶, m, n, p, Q and z are as defined in claim 9.

19. A pharmaceutical composition comprising a therapeutically effective amount of the compound of formula (I) or the stereoisomer, tautomer, mesomer, racemate, enantiomer, diastereomer thereof, or mixture thereof, or the pharmaceutically acceptable salt thereof according to any one of claims 1 to 15, and one or more pharmaceutically acceptable carriers, diluents or excipients.

20. Use of the compound of formula (I) or the stereoisomer, tautomer, mesomer, racemate, enantiomer, diastereomer thereof, or mixture thereof, or the pharmaceutically acceptable salt thereof according to any one of claims 1 to 15 or the pharmaceutical composition according to claim 19 in the preparation of a medicament for inhibiting ERK.

21. Use of the compound of formula (I) or the stereoisomer, tautomer, mesomer, racemate, enantiomer, diastereomer thereof, or mixture thereof, or the pharmaceutically acceptable salt thereof according to any one of claims 1 to 15 or the pharmaceutical composition according to claim 19 in the preparation of a medicament for the treatment or prevention of cancer, inflammation, or other proliferative diseases, and preferably cancer; wherein the cancer is selected from the group consisting of melanoma, liver cancer, kidney cancer, lung cancer, nasopharyngeal cancer, colorectal cancer, colon cancer, rectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma, astrocytoma and glioma.
